# FASCICULE DE BREVET EUROPEEN

(11) **EP 1 210 604 B1**
(45) Date de publication et mention de la délivrance du brevet: **16.04.2008**
(21) Numéro de dépôt: 00948068.2
(22) Date de dépôt: 30.06.2000
(51) Int. Cl.: G01N 33/68, C07K 16/18

(54) **METHODES POUR LE DIAGNOSTIC D'UNE PATHOLOGIE SYNOVIALE OU OSTEOARTICULAIRE**
VERFAHREN ZUR DIAGNOSE VON SYNOVIALEN ODER OSTEOARTIKULÄREN ERKRANKUNGEN
METHODS AND KITS FOR DIAGNOSING OR MONITORING A SYNOVIAL OR OSTEOARTICULAR PATHOLOGY COMPRISING THE USE OF A SPECIFIC MARKER OF THE SYNOVIAL TISSUE DEGRADATION

(30) Priorité: 01.07.1999 FR 9908502
(43) Date de publication de la demande: 05.06.2002
(73) Titulaire: INSTITUT NATIONAL DE LA SANTE ET DE LA RECHERCHE MEDICALE (INSERM), 75654 Paris Cédex 13 (FR)
(72) Inventeur: DELMAS, Pierre, F-69003 Lyon (FR); GARNERO, Patrick, F-69100 Villeurbanne (FR); GINEYTS, Evelyne, F-69004 Lyon (FR)
(74) Mandataire: Desaix, Anne
(86) Numéro de dépôt international: PCT/FR2000/001878
(87) Numéro de publication internationale: WO 2001/002864

(56) Documents cités:
- WO-A-89/12824
- US-A- 5 607 862
- US-A- 5 702 909
- RICARD-BLUM, S. (1) ET AL: "Detectable levels of pyridinoline are present in synovial fluid from various patients with knee effusion: Preliminary results." EUROPEAN JOURNAL OF CLINICAL INVESTIGATION, (1995) VOL. 25, NO. 6, PP. 438-441., XP000892285
- DATABASE BIOSIS [en ligne] BIOSCIENCES INFORMATION SERVICE, PHILADELPHIA, PA, US; WOTTON, S. F. (1) ET AL: "Type IX collagen immunoreactive peptides in synovial fluids from arthritis patients." retrieved from STN XP002132753 & RHEUMATOLOGY (OXFORD), (APRIL, 1999) VOL. 38, NO. 4, PP. 338-345.,
- DATABASE MEDLINE [en ligne] US NATIONAL LIBRARY OF MEDICINE (NLM), BETHESDA, MD, US; SINIGAGLIA L ET AL: "Urinary and synovial pyridinium crosslink concentrations in patients with rheumatoid arthritis and osteoarthritis." retrieved from STN Database accession no. 95216979 XP002132754 & ANNALS OF THE RHEUMATIC DISEASES, (1995 FEB) 54 (2) 144-7.,

## Description

La présente invention concerne un marqueur spécifique de la dégradation du tissu synovial ainsi que des méthodes mettant, en oeuvre ce marqueur dans le diagnostic, le suivi et la détermination d'un pronostic de pathologies synoviales.

Les pathologies ostéoarticulaires ou pathologies des articulations se caractérisent par une dégradation, une destruction ou une résorption des tissus de l'articulation. Lorsque les symptômes radiologiques et cliniques apparaissent, l'articulation a déjà subi des modifications sévères dues à l'atteinte du cartilage et de la matrice osseuse. On cherche à l'heure actuelle un marqueur traduisant un stade plus précoce des pathologies ostéoarticulaires. Dans ce contexte, les inventeurs ont mis en avant le fait que le tissu synovial est le premier dégradé dans certaines pathologies ostéoarticulaires. Ainsi, dans la polyarthrite rhumatoide, le tissu synovial est le premier dégradé, puis le tissu cartilagineux commence à être dégradé, le tissu osseux étant le dernier à être dégradé.

L'identification de marqueurs spécifiques de la dégradation du tissu synovial présente donc un intérêt majeur. En effet, le tissu synovial pouvant être l'un des premiers tissus à être atteint dans une pathologie ostéoarticulaire, un marqueur spécifique de pathologie synoviale peut donc constituer un marqueur précoce de pathologie ostéoarticulaire ou encore un marqueur pré-symptomatique de pathologie ostéoarticulaire.

A ce jour, aucun marqueur spécifique synovial n'a été décrit dans la littérature. L'invention fournit pour la première fois un marqueur spécifique de tissu synovial et plus particulièrement un marqueur de la dégradation du collagène synovial. L'invention fournit un marqueur spécifique dont le niveau reflète à lui seul le niveau de dégradation du collagène synovial permettant ainsi le diagnostic ou le suivi d'évolution de pathologies synoviales. Etant donné que la dégradation du tissu synovial survient aussi lors de pathologies ostéoarticulaires, le niveau de ce marqueur permet également le diagnostic ou le suivi d'évolution de pathologies ostéoarticulaires. Enfin, comme les inventeurs ont qualifié la dégradation du tissu synovial de phase précoce de pathologies ostéoarticulaires, ce marqueur est également un marqueur pré-symptômatique de pathologies ostéoarticulaires.

De préférence, un marqueur spécifique de pathologie synoviale selon l'invention est la pyridinoline glycosylée et plus particulièrement la pyridinoline diglycosylée (Pyr-Gal-Glc), aussi appelée glucosyl-galactosyl-pyridinoline ou pyridinoline disaccharidique. En effet, les présents inventeurs ont identifié le Pyr-Gal-Glc comme étant un marqueur spécifique de la dégradation du collagène synovial.

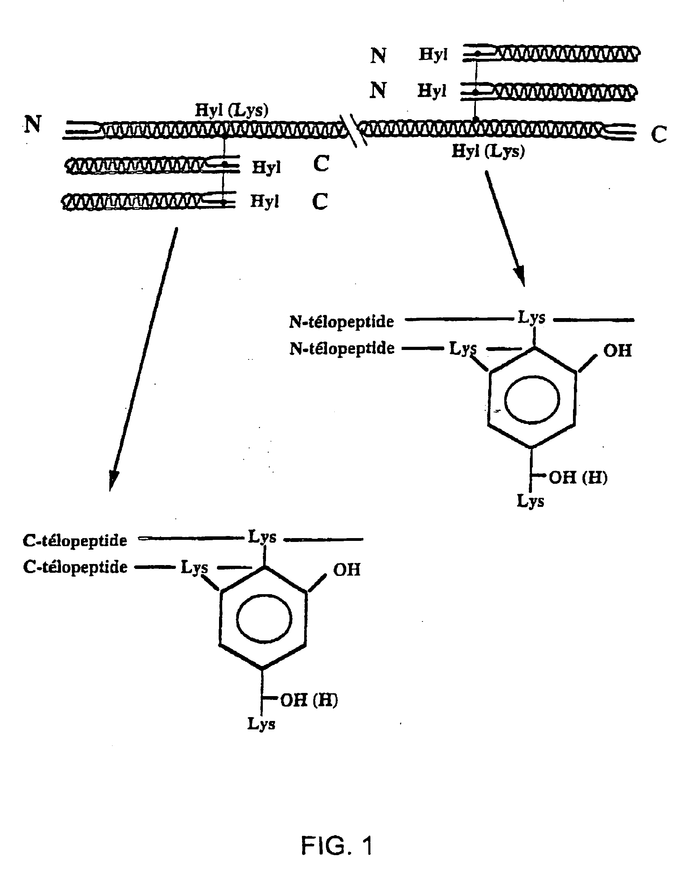

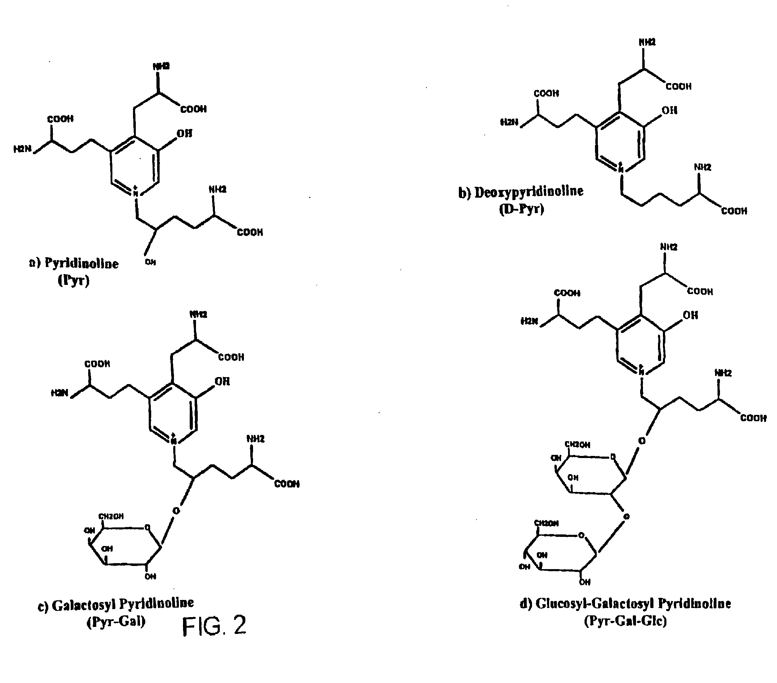

La molécule de pyridinoline résulte de la condensation de trois résidus hydroxylysine dont l'un provient de la région hélicoïdale de la molécule de collagène (type majoritaire : type I pour le tissu osseux, type I et III pour la synoviale, type II pour la cartilage) et les deux autres sont issus des télopeptides C et N terminaux (voir figure 1). Les résidus d'hydroxylysine peuvent être glycosylés par l'addition de sucre au niveau du groupement hydroxyle. Ainsi, si la formation de pyridinoline fait intervenir un résidu hydroxylysine glycosylé, une pyridinoline glycosylée sera produite (voir figure 2 c) et d)). Au contraire, la déoxypyridinoline (voir figure 2 b)) qui est formée par l'intermédiaire d'un résidu lysine ne pourra pas être glycosylée à cause de l'absence de groupement hydroxyle sur la chaîne latérale qui comprend le groupement hydroxyle dans la forme pyridinoline.

Ricard-Blum et al., Detectable levels of pyridinoline are present in synovial fluid from various patients with knee effusion: Preliminary results. European Journal of Clinical Investigation 1995; vol. 25, No. 6, 438-441, révèle la détermination de la présence de pyridinoline originaire du cartilage, des os et de la synoviale dans le liquide synoviale par HPLC.

La pyridinoline diglycosylée a été mesurée dans les urines de patients atteints d'ostéoporose (Robins SP et al ; 3rd int. Symp. On Osteoroposis, Copenhagen, P. 465, ed. by C. Christiansen and K. Overgaard, 1990).

Il a par ailleurs été suggéré d'utiliser la pyridinoline diglycosylée comme indice de résorption du collagène osseux et du collagène cartilagineux et de mesurer à la fois la pyridinoline diglycosylée et la pyridinoline monoglycosylée comme indicateur de pathologie arthritique (demande de brevet n° WO 89/12824).

L'invention a montré que la pyridinoline diglycosylée n'était pas un indice de résorption du collagène osseux ou du collagène cartilagineux mais un indice de pathologie synoviale et que l'augmentation de la pyridinoline diglycosylée dans les liquides biologiques était un indice de pathologie ostéoarticulaire mais pas de pathologie osseuse.

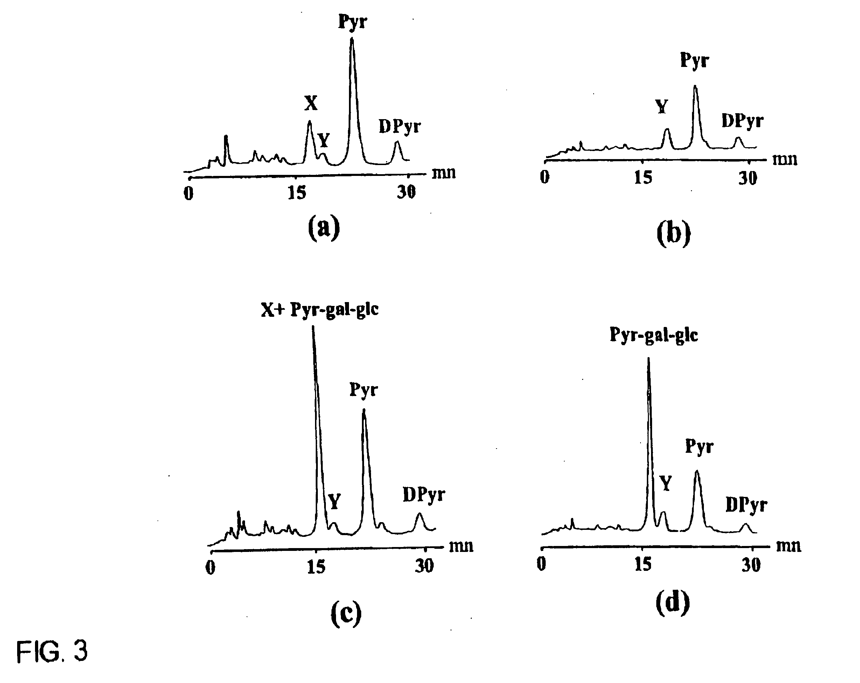

Dans le cadre de l'invention, les inventeurs ont caractérisé le type de glycosylation majoritaire de la pyridinoline dans la matrice osseuse, dans le cartilage et la synoviale (voir figures 3 à 7). Ainsi, les différentes formes de pyridinoline ont été recherchées dans le tissu osseux, cartilagineux et synovial dans un modèle ex-vivo de dégradation tissulaire.

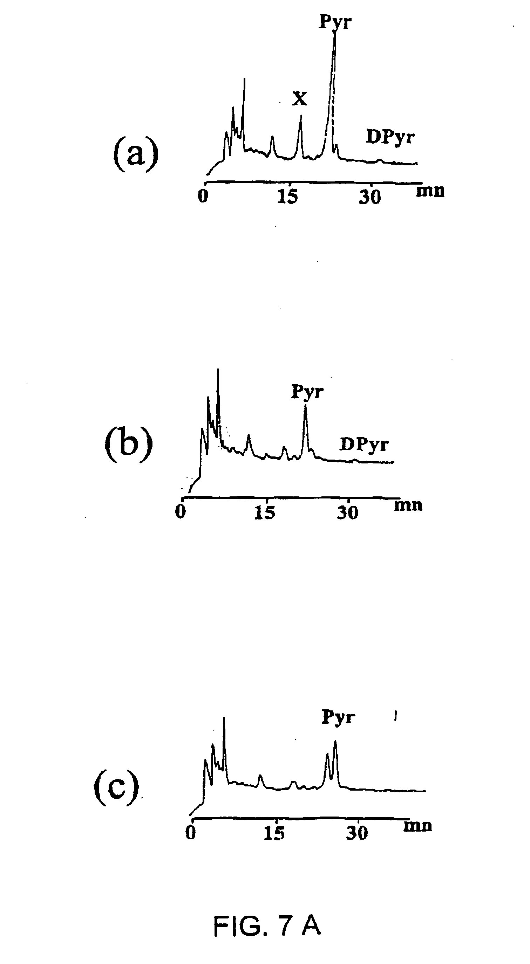

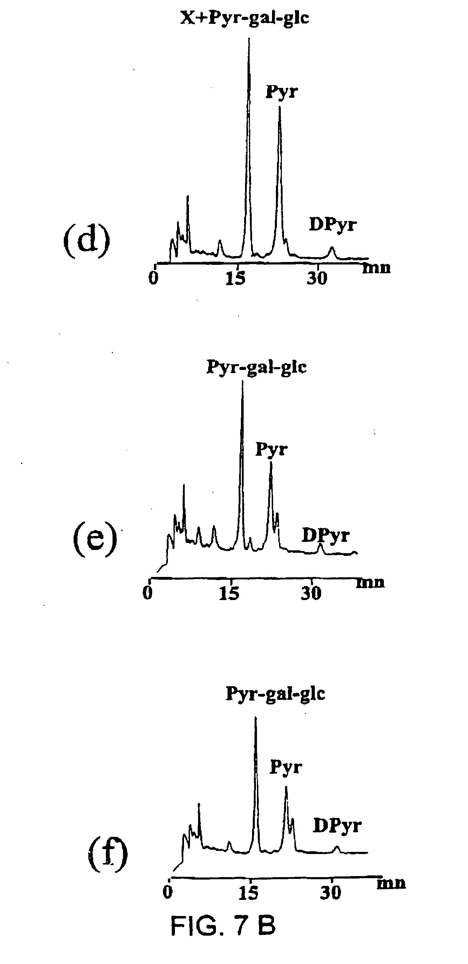

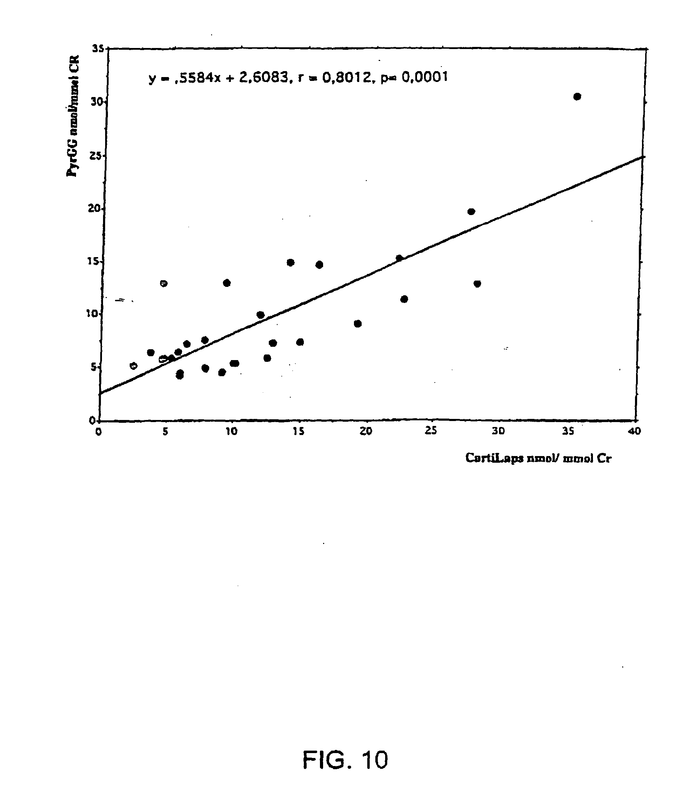

Dans le cadre de l'invention, il a aussi été montré l'existence des formes glycosylées de la pyridinoline dans les urines et l'intérêt de leur dosage pour l'étude des pathologies ostéoarticulaires telles que la polyarthrite rhumatoide ou l'arthrose. La variation de la proportion de chaque forme de la pyridinoline et de la déoxypyridinoline (totale, libre, mono- ou di-glycosylée) dans les pathologies « modèles », c'est à dire caractérisées soit par une augmentation spécifique et intense du remodelage osseux (maladie de Paget), soit par une inflammation de la synoviale avec atteintes cartilagineuses (arthrose, polyarthrite rhumatoide) a ainsi été analysée. L'invention a montré pour la première fois un lien direct entre inflammation et dégradation de la synoviale. Les pyridinolines et déoxypyridinolines totales sont mesurées après hydrolyse acide. Les pyridinolines et déoxypyridinolines libres sont mesurées après hydrolyse alcaline pour les tissus et sans hydrolyse pour les surnageants. Ces résultats sont rapportés aux figures 3 et 7. Ces résultats ont ainsi montré que le marqueur pyridinoline diglycosylée (Pyr-Gal-Glc) était spécifique de pathologie synoviale ou de la dégradation de la synoviale et qu'il était corrélé à un marqueur connu (CartiLaps) spécifique de la pathologie du cartilage ou de la dégradation du cartilage (voir figure 10).

De plus, dans le cadre de l'invention il a été montré l'intérêt du dosage des formes glycosylées de la pyridinoline et plus particulièrement de la forme diglycosylée dans les urines pour le suivi de l'évolution de pathologies ostéoarticulaires (voir figures 8 et 9). Ainsi, plus l'individu est avancé dans la pathologie ou caractérisé par une forme grave de la pathologie, plus le marqueur Pyr-Gal-Glc est mesuré en quantité élevée dans les urines corrélativement à ce qui peut être mesuré avec un marqueur connu tel que le CartiLaps. En effet, la quantité de Pyr-Gal-Glc mesurée chez un individu au stade destructeur est plus élevée que celle mesurée chez un individu au stade non destructeur (figure 11). De plus, chez un individu atteint d'une pathologie ostéoarticulaire, une quantité élevée de Pyr-Gal-Glc dans les urines indique un risque augmenté d'évolution vers un stade de destruction rapide des articulations (figure 13).

La mesure de l'excrétion urinaire des deux formes de glycosylation de la pyridinoline constitue des marqueurs spécifiques de la dégradation de la matrice osseuse, du cartilage et de la synoviale. La spécificité du tissu osseux, du cartilage et de la synoviale par rapport à la peau est apportée par le noyau pyridinolique (le collagène de type I de la peau n'est que très peu ponté par la pyridinoline) et la spécificité tissulaire (synoviale, cartilage, os) par la glycosylation et le type de glycosylation. Une augmentation de la quantité de pyridinoline disaccharidique correspond à une élévation de la dégradation du collagène d'origine synoviale, à une inflammation de la synoviale ou à une prolifération du collagène d'origine synoviale ; une augmentation de la quantité de pyridincline monosaccharidique correspond à une dégradation du collagène d'origine osseuse, tandis que l'augmentation de la quantité de pyridinoline et l'absence de pyridinoline glycosylée correspond à une dégradation du collagène d'origine cartilagineuse.

### Définitions:

- La pyridinoline diglycosylée est désignée par l'abréviation Pyr-Gal-Glc et est aussi appelée glucoeyl-galactosyl-pyridinoline ou pyridinoline disaccharidique.
- un « individu », peut appartenir à une ou plusieurs des catégories suivantes :
   - individu susceptible d'être atteint d'une pathologie synoviale
   - un modèle pour une pathologie donnée et notamment un modèle utilisé dans les études précliniques
   - un être humain
   - un malade
   - un patient ou un individu qui subit un traitement médical ou une opération chirurgicale
   - un individu qui est ou a été sous traitement pour une pathologie ostéoarticulaire
   - un individu atteint d'une pathologie synoviale ou soupçonné d'être atteint d'une telle pathologie
   - un individu atteint d'une pathologie ostéoarticulaire
   - un individu qui n'est pas atteint d'ostéoporose
   - un individu en période de croissance
   - un individu de sexe masculin
   - un individu de sexe féminin
   - un enfant
   - un adulte
   - un individu de sexe féminin pré- ou post-ménopausé
   - un animal tel qu'un rat, une souris, un lapin, un mouton, un primate ou un cheval de course ;
- un « marqueur spécifique de pathologie synoviale » signifie une substance ou un composé qui permet de distinguer une pathologie synoviale d'autres pathologies et notamment d'une atteinte de l'os et/ou du cartilage. De préférence le marqueur spécifique de pathologie synoviale est un marqueur de la dégradation du collagène,synovial.
- une « pathologie synoviale » signifie une augmentation du turn-over, une prolifération, une dégradation, une inflammation, une destruction, une décomposition, un remodelage pathologique ou une dégradation de la synoviale ou du collagène synovial ;
- un « collagène synovial » signifie un collagène de type I, III, IV, V, ou VI, ou un mélange de collagènes choisis parmi lesdits collagènes. De préférence, il s'agit d'un collagène de type majoritaire I et III ;
- une « pathologie ostéoarticulaire » signifie une pathologie d'une ou de plusieurs articulations ou qui implique une prolifération de la synoviale et une atteinte du cartilage. Une pathologie ostéoarticulaire peut être un rhumatisme inflammatoire, une arthropathie métabolique ou un rhumatisme dégénératif, une polyarthrite rhumatoïde, une spondylarthropathie, la goutte, la chondrocalcinose ou l'arthrose.
- un « échantillon biologique » signifie un liquide corporel. Le liquide corporel est généralement extrait de l'individu avant la mise en oeuvre d'une méthode selon l'invention. Le liquide corporel peut être choisi parmi le sang, le sérum, le plasma, l'urine, la salive, la sueur ou le liquide synovial. Le liquide synovial est avantageux dans la mesure où il est spécifique du tissu synovial ou de la synoviale. Un échantillon biologique peut être un tissu extrait de l'individu. Le tissu peut être mis en culture avant que l'on effectue la mesure ou la détection sur ce tissu. Le tissu peut être la synoviale.
- Le « stade destructeur » (destructive) d'une pathologie ostéoarticulaire correspond au stade auquel la destruction ou la dégradation des tissus articulaires peut être visualisée par radiographie, par Résonance Magnétique ou par Résonance Magnétique Nucléaire. Le stade non destructeur (non destructive) correspond au stade auquel la destruction ou la dégradation des tissus articulaires n'est pas encore visualisable par radiographie, par Résonance Magnétique ou par Résonance Magnétique Nucléaire.
- La « pyridinoline libre » signifie pyridinoline liée à aucun peptide
- La « pyridinoline glycosylée » signifie la pyridinoline portant des groupements glycosylés. « Pyridinoline glycosylée » comprend à la fois la pyridinoline glycosylée portant des résidus peptidiques et la pyridinoline glycosylée exempte de peptide. Selon la technique mise en oeuvre pour mesurer la pyridinoline glycosylée, et selon la technique mise en oeuvre dans la préparation de l'échantillon biologique (hydrolyse alcaline ou absence d'hydrolyse), la méthode de l'invention peut mesurer exclusivement la pyridinoline glycosylée libre, ou alternativement la pyridinoline-glycosylée totale.

Les méthodes selon l'invention décrites dans la suite du texte peuvent être des techniques qualitatives ou quantitatives ou à la fois quantitatives et qualitatives. Ainsi, les méthodes décrites dans la suite du texte où l'on mesure une variation de la quantité d'un marqueur spécifique peuvent être des méthodes quantitatives. Les méthodes décrites dans la suite du texte où l'on détecte la présence d'un marqueur spécifique peuvent être des méthodes qualitatives.

De manière générale, l'invention concerne une méthode pour le suivi de la dégradation du collagène synovial chez un individu. La dégradation du collagène synovial peut être normale (c'est à dire non pathologique, dans le cadre du turnover synovial normal) ou pathologique. Les méthodes de l'invention s'appliquent de façon particulièrement avantageuse dans le suivi, le diagnostic et le pronostic de pathologies synoviales ainsi que dans l'évaluation de l'efficacité de traitements thérapeutiques.

Plus particulièrement, l'invention concerne une méthode pour le diagnostic, éventuellement précoce, ou le suivi de l'évolution d'une pathologie ostéoarticulaire impliquant la dégradation de collagène synovial. Cette méthode est caractérisée par :
i) la mise en contact in vitro d'un échantillon biologique provenant d'un individu avec un moyen pour mesurer un marqueur spécifique du niveau de dégradation du collagène synovial;
ii) la détermination du niveau du marqueur spécifique. De préférence le marqueur est un marqueur dont le niveau reflète à lui seul le niveau de dégradation du collagène synovial.

Selon un premier aspect, l'invention concerne une méthode pour le diagnostic ou le suivi de l'évolution d'une pathologie synoviale caractérisée par :
i) la mise en contact in vitro d'un échantillon biologique provenant d'un individu avec un moyen pour mesurer un marqueur spécifique de pathologie synoviale ;
ii) la détermination du niveau du marqueur spécifique
iii) la comparaison du niveau du marqueur avec un niveau de référence représentant l'absence de la pathologie ou représentant un stade prédéterminé de la pathologie, le niveau du marqueur par rapport au niveau de référence indiquant la présence ou l'évolution de la pathologie synoviale.

Une méthode selon cet aspect de l'invention permet de déterminer une évolution positive ou négative dans une pathologie synoviale ainsi que de détecter la survenue ou la fin d'une telle pathologie.

L'individu préféré selon le premier aspect de l'invention est atteint de pathologie synoviale ou susceptible d'être atteint de pathologie synoviale, c'est à dire présentant ou non des symptômes d'une pathologie synoviale.

Le marqueur spécifique de pathologie synoviale préféré est la pyridinoline diglycosylée.

Dans l'application diagnostic de la méthode selon le premier aspect de l'invention, le niveau de référence représentant l'absence de la pathologie ou représentant un stade prédéterminé de la pathologie, peut être choisi en fonction de la valeur moyenne ou de la valeur maximale mesurée pour le marqueur spécifique dans un échantillon d'individus qui ne sont pas atteints d'une pathologie.

Le choix du niveau de référence pour l'application diagnostic de la méthode selon le premier aspect de l'invention peut être déterminé par l'homme du métier en fonction du marqueur spécifique de pathologie synoviale choisi ainsi que de la technique utilisée pour mesurer le marqueur spécifique de pathologie synoviale.

Si le marqueur choisi est la pyridinoline diglycosyléé et la technique est l'HPLC, le niveau de référence qui délimite l'état pathologique de l'état non pathologique peut être compris dans l'intervalle de valeurs comprises entre environ 5 (nmoles/mmoles Créatinine) et environ 9 (nmoles/mmoles Créatinine).

Dans l'application de la méthode de suivi de l'évolution d'une pathologie synoviale selon le premier aspect de l'invention, le niveau de référence peut correspondre à la limite entre l'état non pathologique et l'état pathologique pour une pathologie synoviale ou à la limite entre deux stades distincts de l'évolution de la pathologie synoviale. Deux stades distincts de l'évolution d'une pathologie synoviale peuvent être le stade non-destructeur et le stade destructeur.

La limite entre deux stades distincts de l'évolution d'une pathologie synoviale peut être choisie dans l'intervalle de valeurs comprises entre la valeur moyenne plus un écart-type et la valeur moyenne plus trois écart-types mesurés dans un échantillon contrôle représentatif d'individus non pathologiques qui ne sont pas atteints de pathologie synoviale. Une valeur particulièrement pertinente est celle de la valeur moyenne plus deux écart-types.

La limite entre deux stades distincts de l'évolution d'une pathologie synoviale peut être choisie dans l'intervalle de valeurs comprises entre la valeur moyenne plus un écart-type et la valeur moyenne plus trois écart-types mesurés dans un échantillon représentatif d'individus qui sont au stade le moins avancé parmi les deux stades distincts de l'évolution d'une pathologie synoviale. Une valeur particulièrement pertinente est celle de la valeur moyenne plus deux écart-types.

Le niveau de référence du marqueur spécifique peut être le niveau mesuré antérieurement chez le même individu.

Le choix du niveau de référence pour le suivi de l'évolution d'une pathologie synoviale selon le premier aspect de l'invention peut être déterminé par l'homme du métier en fonction du marqueur spécifique de pathologie synoviale choisi et de la technique utilisée pour mesurer ledit marqueur spécifique.

Si le marqueur choisi est la pyridinoline diglycosylée et la technique est l'HPLC, le niveau de référence qui délimite deux stades distincts de la pathologie et notamment la stade non destructeur du stade destructeur peut être compris dans l'intervalle de valeurs comprises entre environ 5 (mnol/mmol Créatinine) et environ 9 (nmol/mmol Créatinine).

L'invention concerne aussi un kit pour le diagnostic ou le suivi de l'évolution d'une pathologie synoviale caractérisé en ce qu'il comprend au moins un moyen pour mesurer un marqueur spécifique de pathologie synoviale et une mention du niveau de référence représentant l'absence de la pathologie ou représentant un stade prédéterminé de la pathologie.

Selon une variante préférée de l'invention la pathologie synoviale est une pathologie ostéoarticulaire. Selon ce second aspect, l'invention concerne une méthode pour le suivi de l'évolution d'une pathologie ostéoarticulaire caractérisée par :
i) la mise en contact in vitro d'un échantillon biologique provenant d'un individu, avec un moyen pour mesurer un marqueur spécifique de pathologie synoviale ;
ii) la détermination du niveau du marqueur spécifique;
iii) la comparaison du niveau du marqueur avec un niveau de référence représentant un stade prédéterminé de la pathologie, le niveau du marqueur par rapport au niveau de référence indiquant l'évolution de la pathologie ostéoarticulaire.

Une méthode selon le second aspect de l'invention permet de déterminer une évolution positive ou négative dans une pathologie ostéoarticulaire ainsi que de détecter la survenue ou la fin d'une telle pathologie.

De préférence selon cet aspect, l'individu est déjà atteint d'une pathologie ostéoarticulaire.

L'invention concerne aussi un kit pour le suivi de l'évolution d'une pathologie ostéoarticulaire caractérisé un ce qu'il comprend au moins un moyen pour mesurer un marqueur spécifique de pathologie synoviale et une mention du niveau de référence représentant l'absence de la pathologie ou représentant un stade prédéterminé de la pathologie.

Selon cette variante, le marqueur spécifique de pathologie synoviale préféré est aussi la pyridinoline diglycosylée.

Le niveau de référence, pour la méthode et le kit selon le second aspect de l'invention, peut correspondre à la limite entre l'état non pathologique et l'état pathologique ou à la limite entre deux stades distincts de l'évolution de la pathologie. Deux stades distincts de l'évolution d'une pathologie ostéoarticulaire peuvent être le stade non-destructeur et le stade destructeur.

La limite entre le stade pathologique et le stade non-pathologique d'une pathologie ostéoarticulaire peut être choisie dans l'intervalle de valeurs comprises entre la valeur moyenne plus un écart-type et la valeur moyenne plus trois écart-types mesurés dans un échantillon contrôle représentatif d'individus non pathologiques ou qui ne sont pas atteints de pathologie ostéoarticulaire. Une valeur particulièrement pertinente est celle de la valeur moyenne plus deux écart-types.

La limite entre deux stades distincts de l'évolution d'une pathologie ostéoarticulaire peut être choisie dans l'intervalle de valeurs comprises entre la valeur moyenne plus un écart-type et la valeur moyenne plue trois écart-types mesurés dans un échantillon contrôle représentatif d'individus non pathologiques ou qui ne sont pas atteints de pathologie ostéoarticulaire. Une valeur particulièrement pertinente est celle de la valeur moyenne plus deux écart-types.

La limite entre deux stades distincts de l'évolution d'une pathologie ostéoarticulaire peut être choisie dans l'intervalle de valeurs comprises entre la valeur moyenne plus un écart-type et la valeur moyenne plus trois écart-types mesurés dans un échantillon représentatif d'individus qui sont au stade le moins avancé parmi les deux stades distincts de l'évolution d'une pathologie oatéoarticulaire. Une valeur particulièrement pertinente est celle de la valeur moyenne plus deux écart-types.

Le niveau de référence, pour la méthode et le kit selon le second aspect de l'invention, peut être le niveau mesuré antérieurement chez le même individu.

Le choix du niveau de référence, pour la méthode est le kit selon le second aspect de l'invention, peut être déterminé par l'homme du métier en fonction du marqueur spécifique de pathologie synoviale choisi et de la technique utilisée pour mesurer ledit marqueur spécifique.

Si le marqueur choisi est la pyridinoline diglycosylée et la technique est l'HPLC, le niveau de référence qui délimite l'état non pathologique de l'état pathologique peut être compris entre environ 5 (nmol/mmol Créatinine) et environ 9 (nmol/mmol Créatinine.

Si le marqueur choisi est la pyridinoline diglycosylée et la technique est l'HPLC, le niveau de référence qui délimite deux stades distincts de l'évolution d'une pathologie ostéoarticulaire peut être compris entre environ 5 (nmol/mmol Créatinine) et environ 9(nmol/mmol Créatinine).

Selon un troisième aspect, l'invention concerne une méthode pour la détermination du pronostic d'évolution vers une pathologie ostéoarticulaire ou vers un stade de pathologie ostéoarticulaire. En effet, les inventeurs ont constaté que le niveau d'un marqueur spécifique de dégradation synoviale par exemple la pyridinoline diglycosylée chez un individu peut indiquer par rapport à un niveau de référence prédéterminé, le risque que comporte cet individu de subir une destruction accélérée des articulations. Cette méthode se caractérise par :
i) la mise en contact in vitro d'un échantillon biologique prévenant d'un individu avec un moyen pour mesurer un marqueur spécifique de pathologie synoviale ;
ii) la détermination du niveau du marqueur spécifique ;
iii) la comparaison du niveau du marqueur avec un niveau de référence et la déduction d'un pronostic d'évolution vers une pathologie ostéoarticulaire ou vers un stade de pathologie ostéoarticulaire.

Un individu préféré selon le troisième aspect de l'invention est atteint de pathologie ostéoarticulaire ou susceptible d'être atteint de pathologie ostéoarticulaire.

Pour cet aspect de l'invention, le marqueur spécifique de pathologie synoviale préféré est la pyridinoline diglycosylée.

L'invention concerne aussi un kit de diagnostic pour la détermination d'un pronostic d'évolution vers une pathologie ostéoarticulaire ou vers un stade de pathologie ostéoarticulaire, caractérisé en ce qu'il comprend au moins un moyen pour mesurer un marqueur spécifique de pathologie synoviale et une mention du niveau de référence représentant un pronostic.

Une méthode ou un kit selon le troisième aspect de l'invention permet de donner un pronostic d'évolution vers une pathologie ostéoarticulaire pour un individu qui ne présente pas de pathologie ostéoarticulaire ou vers un stade de pathologie ostéoarticulaire, notamment le stade destructeur, pour un individu qui est au stade non destructeur de pathologie ostéoarticulaire.

Le pronostic déterminable par une méthode ou un kit selon le troisième aspect de l'invention peut notamment couvrir une période de 1, 2, 3, 4 ou 5 ans.

Le niveau de référence pour une méthode ou un kit selon le troisième aspect de l'invention peut être déterminé par une étude de suivi longitudinal d'un échantillon d'individus à risque pour une pathologie ostéoarticulaire ou d'individus à un stade non destructeur de pathologie osteoarticulaire. Le niveau de référence de la méthode décrite ci-dessus peut aussi être déterminé par l'analyse d'échantillons existants extraits à différents temps pour un même individu.

Selon un quatrième aspect, l'invention concerne une méthode pour la détermination de l'efficacité d'un médicament admintstré à un individu pour le traitement d'une pathologie ostéoarticulaire caractérisé par :
i) la mise en contact in vitro d'un échantillon biologique provenant d'un individu sous traitement, avec un moyen pour mesurer un marqueur spécifique de pathologie synoviale ;
ii) la détermination du niveau du marqueur spécifique :
iii) la comparaison du niveau du marqueur avec un niveau de référence représentant un stade prédéterminé de la pathologie, le niveau du marqueur par rapport au niveau de référence indiquant l'évolution de la pathologie synoviale, et ainsi le degré d'efficacité du traitement

un individu selon le quatrième aspect de l'invention est de préférence un animal utilisé comme modèle pour l'étude préclinique de l'efficacité de traitement d'une pathologie ostéoarticulaire donnée, ou peut être un être humain dont le traitement est suivi.

L'invention concerne aussi un kit pour la détermination de l'efficacité d'un médicament administré à un individu pour le traitement d'une pathologie ostéoarticulaire, caractérisé en ce qu'il comprend au moins un moyen pour mesurer un marqueur spécifique de pathologie synoviale et une mention du niveau de référence représentant un niveau d'efficacité du médicament.

Pour le quatrième aspect de l'invention, le marqueur spécifique de pathologie synoviale préféré est la pyridinoline diglycosylée.

Le traitement peut être curatif ou prophylactique.

Le niveau de référence selon le quatrième aspect de l'invention peut être le niveau mesuré antérieurement chez le même individu. Le niveau mesuré antérieurement est préférentiellement mesuré avant le début du traitement curatif ou prophylactique d'une pathologie ostéoarticulaire ou avant l'administration du médicament destiné au traitement d'une pathologie ostéoarticulaire.

Le niveau de référence selon le quatrième aspect de l'invention peut correspondre à la limite entre l'état non pathologique et l'état pathologique pour une pathologie ostéoarticulaire ou à la limite entre deux stades distincts de l'évolution de la pathologie ostéoarticulaire. Deux stades distincts de l'évolution d'une pathologie ostéoarticulaire peuvent être le stade non-destructeur et le stade destructeur.

La limite entre le stade pathologique et le stade non pathologique pour une pathologie ostéoarticulaire peut être choisie dans l'intervalle de valeurs comprises entre la valeur moyenne plus un écart-type et la valeur moyenne plus trois écart-types mesurés dans un échantillon contrôle représentatif d'individus non pathologiques ou qui ne sont pas atteints de pathologie ostéoarticulaire. Une valeur particulièrement pertinente est celle de la valeur moyenne plus deux écart-types.

La limite entre deux stades distincts de l'évolution d'une pathologie astéoarticulaire peut être choisie dans l'intervalle de valeurs comprises entre la valeur moyenne plus un écart-type et la valeur moyenne plus trois écart-types mesurés dans un échantillon contrôle représentatif d'individus non pathologiques ou qui ne sont pas atteints de pathologie ostéoarticulaire. Une valeur particulièrement pertinente est celle de la valeur moyenne plus deux écart-types.

La limite entre deux stades distincts de l'évolution d'une pathologie ostéoarticulaire peut être choisie dans l'intervalle de valeurs comprises entre la valeur moyenne plus un écart-type et la valeur moyenne plus trois écart-types mesurés dans un échantillon représentatif d'individus qui sont au stade le moins avancé parmi les deux stades distincts de l'évolution d'une pathologie ostéoarticulaire. Une valeur particulièrement pertinente est celle de la valeur moyenne plus deux écart-types.

Le choix du niveau de référence selon le quatrième aspect de l'invention peut être déterminé par l'homme du métier en fonction du marqueur spécifique de pathologie synoviale choisi et de la technique utilisée pour mesurer ledit marqueur spécifique.

Si le marqueur choisi est la pyridinoline diglycosylée et la technique est l'HPLC, le niveau de référence qui délimite l'état non pathologique de l'état pathologique pour une pathologie ostéoarticulaire peut être compris entre environ 5 (nmol/mmol Créatinine) et environ 9 (nmgl/mml Créatinine).

Si le marqueur choisi est la pyridinoline diglycosylée et la technique est l'HPLC, le niveau de référence qui délimite deux stades distincts de l'évolution d'une pathologie ostéoarticulaire peut être compris entre environ 5 (nmol/mmol Créatinine) et environ 9 (nmol/mmol Créatinine).

L'efficacité du traitement curatif ou prophylactique déduite de la méthode ou du kit selon le quatrième aspect de l'invention peut être positive, nulle ou négative.

Si on déduit de la comparaison du niveau du marqueur mesuré avec un niveau de référence une tendance à l'amélioration de l'état de l'individu étudié, on peut alors conclure que le traitement curatif ou prophylactique utilisé pour cet individu présente au moins un début d'efficacité. L'écart entre le niveau du marqueur mesuré chez l'individu et le niveau de référence permet d'apprécier de manière quantitative ou qualitative le niveau d'efficacité du traitement curatif ou prophylactique testé.

L'efficacité d'un traitement curatif (cure) ou prophylactique (prévention) d'une pathologie ostéoarticulaire peut ainsi être corrélée à une mesure de la variation de la quantité d'un marqueur spécifique de pathologie synoviale.

La présente invention divulge également une méthode de détermination de la toxicité associée à une pathologie ostéoarticulaire ou synoviale d'un médicament destiné au traitement d'une pathologie. En effet, certains médicaments peuvent provoquer des effets secondaires au niveau synovial et notamment peuvent conduire à une dégradation du collagène synovial. Cette méthode est caractérisée par:
i) La mise en contact in vitro d'un échantillon biologique provenant d'un individu avec un moyen pour mesurer un marqueur spécifique de pathologie synoviale,
ii) la mesure du niveau de ce marqueur spécifique,
iii) éventuellement la comparaison du niveau de ce marqueur avec un niveau de référence représentant la présence ou un stade prédéterminé de la pathologie, le niveau du marqueur par rapport au niveau de référence indiquant le degré de la pathologie et ainsi la degré de la toxicité associée à une pathologie synoviale ou ostéoarticulaire.

La description mentionne aussi un kit de diagnostic pour la détermination de la toxicité d'un médicament destiné au traitement d'une pathologie, caractérisé en ce qu'il comprend au moins un moyen pour mesurer un marqueur spécifique de pathologie synoviale et une mention du niveau de référence représentant un niveau de toxicité du médicament.

Par médicament, on entend notamment toute composition pharmaceutique dont la toxicité sur l'individu est susceptible d'être associée ou de s'exprimer par une pathologie synoviale ou une pathologie ostéoarticulaire.

Le niveau de référence peut correspondre à la limite entre l'état non pathologique et l'état pathologique pour une pathologie ostéoarticulaire ou à la limite entre deux stades distincts de l'évolution de la pathologie ostéoarticulaire. Deux stades distincts de l'évolution d'une pathologie ostéoarticulaire peuvent être le stade non-destructeur et le stade destructeur.

La limite entre le stade pathologique et le stade non pathologique pour une pathologie ostéoarticulaire peut être choisie dans l'intervalle de valeurs comprises entre la valeur moyenne plus un écart-type et la valeur moyenne plus trois écart-types mesurés dans un échantillon contrôle représentatif d'individus non pathologiques ou qui ne sont pas atteints de pathologie ostéoarticulaire. Une valeur particulièrement pertinente est celle de la valeur moyenne plus deux écart-types.

La limite entre deux stades distincts de l'évolution d'une pathologie ostéoarticulaire peut être choisie dans l'intervalle de valeurs comprises entre la valeur moyenne plus un écart-type et la valeur moyenne plus trois écart-types mesurés dans un échantillon contrôle représentatif d'individus non pathologiques ou qui ne sont pas atteints de pathologie ostéoarticulaire. Une valeur particulièrement pertinente est celle de la valeur moyenne plus deux écart-types.

-La limite entre deux stades distincts de l'évolution d'une pathologie ostéoarticulaire peut être choisie dans l'intervalle de valeurs comprises entre la valeur moyenne plus un écart-type et la valeur moyenne plus trois écart-types mesurés dans un échantillon représentatif d'individus qui sont au stade le moins avancé parmi les deux stades distincts de l'évolution d'une pathologie ostéoarticulaire. Une valeur particulièrement pertinente est celle de la valeur moyenne plus deux écart-types.

Le niveau de référence peut être le niveau mesuré antérieurement chez le même individu. Le niveau mesuré antérieurement est préférentiellement mesuré avant le début du traitement curatif ou prophylactique ou avant l'administration du médicament.

Le choix du niveau de référence peut être déterminé par l'homme du métier en fonction du marqueur spécifique de pathologie synoviale choisi et de la technique utilisée pour mesurer ledit marqueur spécifique.

Si le marqueur choisi est la pyridinoline diglycosylée et la technique est l'HPLC, le niveau de référence qui délimite l'état non pathologique de l'état pathologique pour une pathologie ostéoarticulaire peut être compris entre environ 5 (nmol/mmol Créatinine) et environ 9 (nmol/mmol Créatinine).

Si le marqueur choisi est la pyridinoline diglycosylée et la technique eut l'HPLC, le niveau de référence qui délimite deux stades distincts de l'évolution d'une pathologie ostéoarticulaire peut être compris entre environ 5 (nmol/mmol Créatinine) et environ 9 (nmol/mmol Créatinine).

La toxicité du traitement curatif ou prophylactique déduite de cette méthode peut exister ou être nulle.

Si on déduit de la comparaison du niveau du marqueur mesuré avec un niveau de référence une apparition de pathologie synoviale ou ostéoarticulaire, on peut alors conclure que le traitement curatif ou prophylactique utilisé pour cet individu présente au moins un début de toxicité. L'écart entre le niveau du marqueur mesuré chez l'individu et le niveau de référence permet d'apprécier de manière quantitative ou qualitative le niveau de toxicité du traitement curatif ou prophylactique testé.

En effet, certains traitements ou médicaments ont pour effet toxique une pathologie synoviale ou une pathologie ostéoarticulaire. La détection de telles pathologies permet donc de détecter la toxicité d'un traitement ou d'un médicament.

La présente description divulge aussi une méthode pour la diagnostic précoce d'une pathologie ostéoarticulaire caractérisé par :
i) la mise en contact in vitro d'un échantillon biologique provenant d'un individu, avec un moyen pour mesurer un marqueur spécifique de pathologie synoviale ;
ii) la détermination du niveau du marqueur spécifique :
iii) éventuellement la comparaison du niveau du marqueur avec un niveau de référence représentant la présence de la pathologie, le niveau au marqueur par rapport au niveau de référence indiquant la présence actuelle ou potentielle de la pathologie synoviale.

Le tissu synovial étant l'un des première atteints dans une pathologie ostéoarticulaire par exemple la polyarthrite rhumatoïde, une telle méthode constitue un moyen de déceler de manière précoce, pré-symptomatique ou avant la phase destructrice la survenue d'une pathologie ostéoarticulaire,

aussi un kit- pour le diagnostic précoce d'une pathologie synoviale caractérisé en ce qu'il comprend au moins un moyen pour mesurer un marqueur spécifique de pathologie synoviale et une mention du niveau de référence représentant l'absence de la pathologie.

Un individu préféré est atteint de pathologie ostéoarticulaire ou susceptible d'être atteint de pathologie ostéoarticulaire.

Le marqueur spécifique de pathologie synoviale préféré est la pyridinoline diglycosylée.

Le niveau de référence peut correspondre à la limite entre l'état non pathologique et l'état pathologique pour une pathologie ostéoarticulaire.

La limite entre le stade pathologique et le stade non pathologique pour une pathologie ostéoarticulaire peut être choisie dans l'intervalle de valeurs comprises entre la valeur moyenne plus un écart-type et la valeur moyenne plus trois écart-types mesurés dans un échantillon contrôle représentatif d'individus non pathologiques ou qui na sont pas atteints de pathologie ostéoarticulaire. Une valeur particulièrement pertinente est celle de la valeur moyenne plus deux écart-types.

Le choix du niveau de référence peut être déterminé par l'homme du métier en fonction du marqueur spécifique de pathologie synoviale choisi ainsi que de la technique utilisée pour mesurer le marqueur spécifique de pathologie synoviale.

Si le marqueur choisi est la pyridinoline diglycosylée et la technique est l'HPLC. le niveau de référence qui délimite l'état non pathologique de l'état pathologique pour une pathologie ostéoarticulaire peut être compris entre environ 5 (nmol/mmol Créatinine) et environ a (nmol/mmol Créatinine).

Dans toutes les méthodes ou kits selon l'invention, la mesure du niveau du marqueur spécifique de pathologie synoviale peut être effectués par une technique inmunologique, par immuno-dosage, par fluorescence, par spectroscopie aux ultraviolets ou encore par détection électrochimique.

Par technique immmologique ou immuno-dosage, on peut entendre une technique utilisant des anticorps spécifiques monoclonaux ou polyclonaux, une technique ELISA, une technique immuno-enzymatique, une technique d'immunofluorescence, une technique radio-immunologique, une technique electrochemo-immunologique ou une technique chemo-immunologique.

Par technique immunologique ou immuno-dosage, on peut entendre l'une quelconque des techniques décrites dans l'ouvrage suivant : Diamandis et Cristopoulos, Immunoassay, Academic Press, San Diego, 1996, notamment les pages 579 et suivantes.

De préférence, la mesure du niveau du marqueur spécifique est réalisée à partir d'un échantillon biologique qui n'a subit aucune hydrolyse acide ou alcaline préalable, particulièrement lorsqu'il s'agit d'urine.

Le marqueur spécifique de pathologie synoviale préféré est la pyridinoline diglycosylée.

De préférence, la pyridinoline glycosylée ou la pyridinoline diglycosylée est liée à un ou plusieurs peptides spécifiques du collagène synovial, du collagène de type I, du collagène de type III, du collagène de type IV, du collagène de type v, du collagène de type VI, du collagène de type I et III ou d'un mélange de collagènes choisis parmi les types I, III. IV, V et VI.

La forme di-glycosylée de la pyridinoline est avantageusement purifiée et caracterisée selon le procédé décrit dans l'exemple 3 de la présente demande.

La glucosyl-galactosyl pyridinoline isolée ou sous forme purifiée peut être utilisée pour produire des anticorps chez un individu.

Un moyen pour mesurer un marqueur spécifique de pathologie synoviale est de préférence un anticorps capable de reconnaître de manière spécifique un marqueur spécifique de pathologie synoviale et notamment la glucosyl-galactosyl pyridinoline.

La description mentionne aussi un anticorps capable de reconnaître de manière spécifique la glucosyl-galactosyl-pyridinoline.

Par «reconnaître de manière spécifique la glucosyl-galactosyl-pyridinoline », on peut entendre la capacité à distinguer la glucosyl-galactosyl pyridinoline das autres dérivés de la pyridinoline qui ne comprennent pas la partie disaccharidique ou la partie glucosyl-galactosyl de la glucosyl-galactosyl-pyridinoline. De préférence de tels anticorps ne présentent pas de réactivité croisée avec des dérivés de la pyridinoline autre que le dérivé diglycosylé.

Les anticorps peuvent être monoclonaux ou polyclonaux et reconnaissent de préférence un ou plusieurs épitopes uniques à la forme diglycosylée de la pyridinoline. Il peut s'agir de pyridinoline diglycosylée libre (sans peptide) ou pyridinoline diglycosylée liée à des résidus peptidiques, ou les deux formes.

La description mentionne des anticorps produits ou susceptibles d'être produits à partir de la glucosyl-galactosyl-pyridinoline isolée ou sous forme purifiée et notamment à partir de la glucosyl-galactosyl-pyridinoline isolée selon l'invention.

Les anticorps définis ci-dessus constituent des moyens préférés pour mesurer un marqueur spécifique de pathologie synoviale.

Les anticorps définis ci-dessus peuvent être obtenus par toute technique connue à ce jour permettant d'obtenir un anticorps à partir d'un marqueur spécifique de pathologie synoviale et notamment de la glucosyl-galactosyl pyridinoline.

L'obtention d'un anticorps spécifique de la glucosyl-galactosyl pyridinoline peut nécessiter que ledit antigène soit dans une première étape extrait et purifié à partir d'urine par exemple. Une telle extraction ou purification peut être réalisée de manière avantageuse par la technique décrite dans l'exemple 3 de la présente demande.

### Légende des figures :

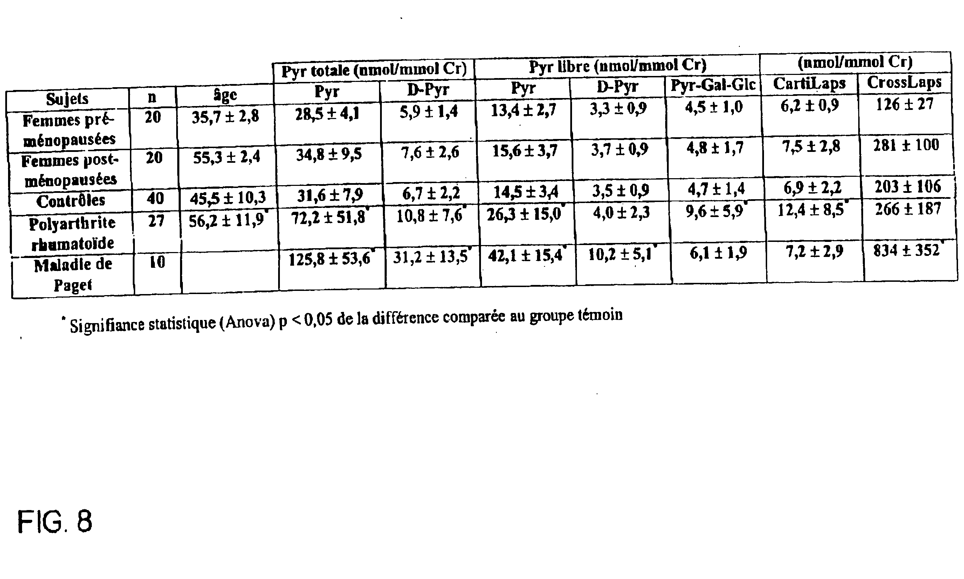

- **Figure 1** **:**
   Représentation schématique des molécules de pyridinoline permettant le pontage du collagène.
- **Figure 2** :
   Forme hydroxy- et deoxy- de la pyridinoline :
   a) Pyridinoline (Pyr) et
   b) b) Déoxypyridinoline (D-Pyr) Formes glycosylées de la pyridinoline (Pyr) :
   e) galactosyl-pyridinoline (Pyr-Gal) et
   d) glucosyl-galactosyl pyridinoline (Pyr-Gal-Glc).
- **Figure 3** :
   Profils de chromatographie de tissu synovial humain (a) et (c) et de tissu osseux humain (b) et (d) traités par hydrolyse alcaline. Pour les profils (c) et (d), du standard Pyr-gal-glc purifié à partir d'urine à été ajouté aux échantillons tissulaires utilisés pour les profils de chromatographie (a) et (b).
   Pyr et D-Pyr représentent les formes hydroxy- et deoxy- de la pyridinoline non glycosylée respectivement.
   Le composé X présent dans le tissu synovial mais pas dans le tissu osseux co-migre avec du standard Pyr-Gal-Glc purifié à partir d'urine.
   Le composé Y présent dans l'os et en quantité très faible dans le tissu synovial ne co-migre pas avec du standard Pyr-Gal-Glc purifié à partir d'urine.
- **Figure 4** **:**
   Profils de chromatographie obtenus à partir de tissu synovial humain (a), de cartilage humain (b) et d'os humain (c) hydrolysés par NaOH 2M pendant 15 heures. Les composés X et Y se transforment en Pyr après hydrolyse acide et le composé X co-migre avec du standard de Pyr-gal-glc purifié à partir d'urine. (d) correspond au profil de chromatographie d'une urine.
   Pyr et D-Pyr représentent les formes hydroxy- et deoxy- de la pyridinoline non glycosylée respectivement.
   Le composé X présent dans le tissu synovial mais pas dans le tissu osseux co-migre avec du standard Pyr-Gal-Glc purifié à partir d'urine.
   Le composé Y présent dans l'os et en quantité très faible dans le tissu synovial ne co-migre pas avec du standard Pyr-Gal-Glc purifié à partir d'urine.
- **Figure 5** :
   Pourcentage mesuré par HPLC sur colonne en phase inverse de Pyr-gal-glc / Pyr libre présent dans le tissu synovial, l'os et le cartilage humains après hydrolyse par NaOH 2M pendant des temps variant de 5 à 20 heures.
   Le symbole « - » signifie non-détectable.
- **Figure 6** :
   Pourcentage mesuré par HPLC sur colonne en phase inverse de Pyr-gal/ Pyr libre présent dans l'os, le cartilage et le tissu synovial humains après hydrolyse par NaOH 2M pendant des temps variant de 5 à 20 heures.
   Le symbole « - » signifie non-détectable.
- **Figure 7** :
   - **Figure 7A** : profils de chromatographie de (a) surnageant de culture de synoviale, (b) surnageant de culture d'os, (c) surnageant de culture de cartilage.
   - **Figure 7B** : profils de chromatographie de (d) surnageant de culture de synoviale supplémenté en standard Pyr-gal-glc, Pyr et D-pyr, (e) surnageant de culture d'os supplémenté en standard Pyr-gal-glc, Pyr et DPyr, (f) surnageant de culture de cartilage supplémenté en standard Pyr-gal-glc, Pyr et DPyr. Le composé X du surnageant de culture de synoviale co-migre avec du standard Pyr-gal-glc purifié à partir d'urine et se transforme en Pyr après hydrolyse acide.
- **Figure 8** :
   Excrétion urinaire des pyridinolines totales (formes libres et peptidiques) et des formes de pyridinoline libres chez des adultes normaux (contrôles) et chez des patients atteints de polyarthrite rhumatoïde ou de la maladie de Paget.
   « Pyr totale » signifie pyridinoline totale ou déoxypyridinoline totale.
   « Pyr libre » signifie pyridinoline libre ou déoxypyridinoline libre.
- **Figure 9** :
   Pourcentage d'augmentation de l'excrétion urinaire des différents marqueurs dans la polyarthrite rhumatoïde (PR) et la maladie de Paget (Paget) par comparaison à un groupe témoin (voir figure 8).
   « PyrGG » signifie Pyr-Gal-Glc ou pyridinoline diglycosylée.
- **Figure 10** :
   Corrélation linéaire entre un marqueur spécifique du cartilage (CartiLaps) et la pyridinoline diglycosylée (PyrGG).
- **Figure 11** :
   Comparaison de la quantité de pyridinoline diglycosylée (Pyr-Gal-Glc) mesurée chez des individus normaux ou des patients atteints de polyarthrite rhumatoïde (PR) à un stade destructeur (AR destructrice) ou non destructeur (PR non destructrice).
- **Figure 12**
   Corrélation entre l'excretion urinaire de Pyr-gal-glc, les indices algofonctionnels de Lequesne et Womac et l'interligne articulaire (espace tibio-fémoral) chez 50 patients avec gonarthrose.
- **Figure 13** **:**
   Corrélation entre le taux de base de Pyr-Gal-Glc urinaire mesuré par HPLC et le risque de destruction rapide des articulations our l'année suivante examiné par radiographie des mains et des pieds aux rayons X chez 112 patients atteints de polyarthrite rhumatoïde depuis moins de trois ans.

### Exemples

### Exemple 1 : étude du type de glycosylation des pyridinolines dans le tissu osseux, cartilagineux et synovial.

### Méthode :

Les tissus humains proviennent de prélèvement d'os, de cartilage et de synoviale recueillis au cours d'interventions chirurgicales pour la mise en place de prothèse de hanche. Les échantillons tissulaires sont finement broyés dans l'azote liquide puis répartis en aliquotes de 10 mg et hydrolysés par NaOH 2M à 110°C. Les temps d'hydrolyse varient entre 5 et 24 heures. Contrairement à l'hydrolyse acide qui libère les sucres liés aux pyridinolines, l'hydrolyse alcaline permet de libérer les molécules de pyridinolines du collagène sans ôter les résidus galactose et glucose éventuellement présents. Après hydrolyse alcaline, les pyridinolines libres sont extraites par chromatographie de partition sur colonne de cellulose. La séparation et la quantification des différentes formes de pyridinolines glycosylées s'effectuent par HPLC sur colonne en phase inverse ultrasphere ODS Beckman (5mm, 25 cm X 4,6 mm) à un débit de 1 ml /minute par une solution à 6% d'acétonitrile et 0.15% d'acide heptafluorobutyrique. Elles sont détectées grâce à leurs fluorescences naturelles à des longueurs d'onde d'excitation et d'émission respectives de 297 nm et 395 nm.

### Résultats :

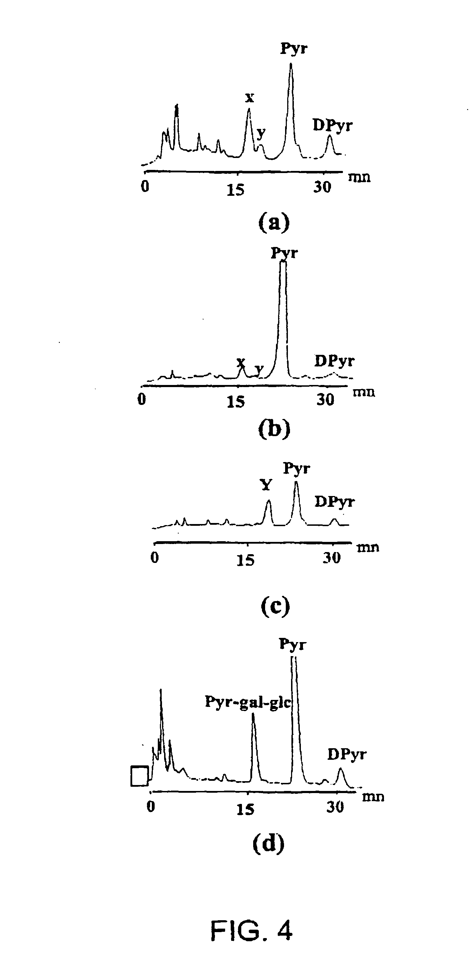

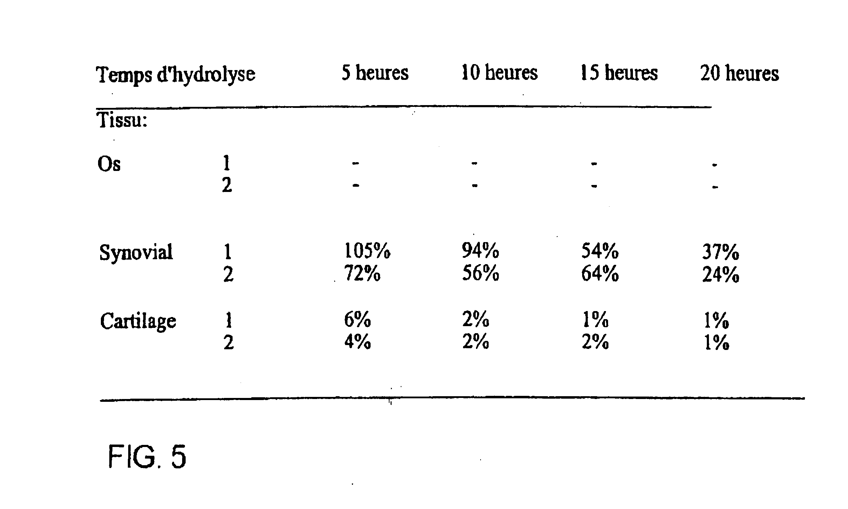

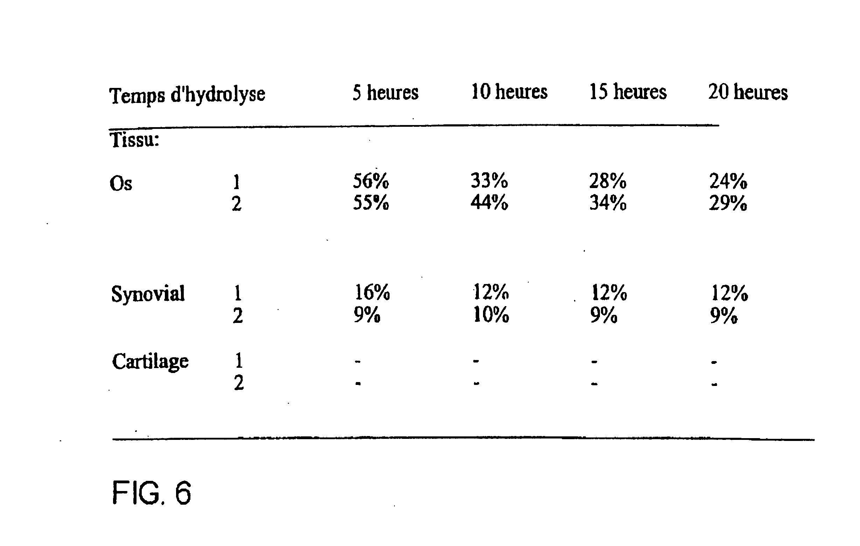

Sur les profils chromatographiques obtenus à partir des hydolysats alcalins de synoviale (figure 3a) et d'os (figure 3b) nous avons pu mettre en évidence la présence de deux composés fluorescents appelés X et Y qui éluent avant la pyridinoline libre non glycosylée (Pyr). Après collecte et hydrolyse acide nous avons pu montrer que ces deux composés se transformaient en pyridinoline libre non glycosylée. Le composé X, présent dans les hydrolysats de tissu synovial et absent des hydrolysats de tissu osseux et co-migre avec du standard de glucosyl-galactosyl pyridinoline (Pyr-gal-glc) purifié à partir d'urine (figure 3c). Le composé Y, présent essentiellement dans les hydrolysats de tissu osseux et élué juste après le standard de Pyr-gal-gLc (figure 3d), correspond à la forme monoglycosylée de la pyridinoline (Pyr-gal). Le cartilage contient peu de pyridinoline glycosylée (figure 4b). En effet après 10 heures d'hydrolyse Pyr-gal-glc ne représente que 2% de la Pyr libre (Figure 5). Le tissu synovial contient essentiellement de la Pyr-gal-glc. Elle représente après 10 heures d'hydrolyse 75% de la Pyr libre (Figure 5). Le tissu osseux contient essentiellement de la Pyr-gal. Elle représente après 10 heures d'hydrolyse 39% de la Pyr libre (Figure 6). Il faut noter également que le % de pyridinolines glycosylées diminue plus l'hydrolyse alcaline est prolongée. Ceci est probablement lié au fait que les glycosylations des pyridinolines sont en partie labiles à l'hydrolyse alcaline. Ces expériences indiquent que le type de glycosylation des molécules de pyridinoline est différent entre l'os, la synoviale et le cartilage. Dans la synoviale la glycosylation est de type gal-glc, celle de l'os est de type gal et le cartilage parait très peu glycosylé.

### Exemple 2 : étude du type de glycosylstion des pyridinolines dans un modèle de culture ex-vivo d'explants tissulaires humains

### Méthodes :

Les prélèvements de tissu osseux, de cartilage et de synoviale, recueillis stérilement au cours des interventions chirurgicales sont placés dans des pots stériles contenant du tampon PBS (Phosphate Buffer Saline) contenant 100 U / ml de pénicilline et 10 ng / ml de streptomycine (Gibco, Eragny, France), puis transportés rapidement au laboratoire. Les trois types de tissu sont alors séparés les uns des autres, puis coupés en cubes d'environ 5 mm de côté (environ 125 mm3) pour la synoviale et l'os, et en lamelles d'environ 5 mm de côté et 1 mm d'épaisseur (environ 25 mm3) pour le cartilage. Les fragments sont alors placés dans des plaques à six puits (Falcon, CLV, Villeurbanne, France) contenant 5 ml de milieu de culture RPMI 1640 (Gibco, Eragny, France) supplémenté avec 2% de sérum de veau foetal (SVF) (Techgen, Les Ullis, France), 2 mM de glutamine (Gibco, Eragny, France), 20 mM de tampon Hépès (Gibco, Eragny, France), 100 U / ml de pénicilline et 10 ng / ml de streptomycine (Techgen, Les Ullis, France). De L'interleukine 1 (IL-1) (Bachem, Bale Biochimie SARL, Voisins-le-Bretonneux, France) est ajoutée dès le premier jour aux cultures d'explants d'os et de synoviale à la concentration de 1 ng/ml de milieu de culture et du plasminogène à la concentration 5 mM est ajouté en plus de l'IL-1 au culture de cartilage afin d'activer le processus de dégradation tissulaire. Les boites sont incubées à 37° C, sous 95% d'humidité et 5% de CO2 pendant sept jours. Après 7 jours de culture, les surnageants sont filtrés (Centrisart Cut-Off 5000, Sartorius) puis analysés par HPLC selon la technique décrite dans l'exemple 1.

### Résultats :

Sur les profils de chromatographie obtenus à partir des surnageants de culture de tissu synovial (figure 7a) nous avons pu mettre en évidence la présence d'un composé fluorescent appelé X qui élue avant la pyridinoline libre non glycosylée et co-migre avec du standard de Pyr-gal-glc purifié à partir d'urine (figure 7d). Après collecte et hydrolyse acide nous avons pu montrer que ce composé se transformait en pyridinoline libre non glycosylée. Ce composé X présent dans les surnageants de culture de tissu synovial est absent des surnageants de culture de tissu osseux (figure 7b et 7e). Un composé co-migrant avec le standard de Pyr-gal-glc est retrouvé seulement sous forme de trace dans les surnageants de cartilage (figure 7c et 7f). Les trop faibles quantités n'ont pas permis de vérifier ou de déceler s'il s'agissait bien d'une forme de la pyridinoline.
Ces expériences confirment les résultats déjà obtenus avec l'hydrolyse alcaline des tissus dans l'exemple 1.

### Exemple 3 : Purification de la forme di-glycosylée de la pyridinoline

La forme diglycosylée (Pyr-gal-glc) de la pyridinoline a été retrouvée en quantité significative dans les urines (figure 4d). Les urines d'enfants (2-13 ans) sont relativement riches en pyridinoline. Nous avons opté pour une purification de la pyridinoline diglycosylée à partir d'urine.

### Méthode :

### - Purification de la pyridinoline diglycosylée :

50 litres d'urine d'enfants ont été récoltés, filtrés et lyophilisés. Le lyophilisât d'urine a été dissout dans de l'eau distillée de façon à obtenir une concentration X 10 des urines. 500 ml d'urines concentrées ont été mélangés à 500 ml d'acide acétique, 2000 ml de n-butanol et 100 g de cellulose en poudre (CF1, Whatman). L'ensemble a été mélangé sous agitation magnétique pendant 5 minutes, puis a été passé sur un entonnoir filtrant retenant la cellulose. La cellulose récupérée par filtration a été lavée par 12 litres d'une solution de lavage contenant 8 litres de n-butanol pour 2 litres d'acide acétique et 2 litres d'eau distillée. L'opération a été réalisée en 12 séries de lavages consécutifs par 1 litre de solution. Après lavage, les molécules de pyridinolines sont éluées par 500 ml d'eau distillée et l'éluat est lyophilisé. Après lyophilisation les éluats ont été dissous dans 10 % d'acide acétique et déposés sur une colonne de gel filtration Sephadex G10 (Pharmacia) de 2 litres. Les fractions contenant la pyridinoline diglycosylée ont été récupérées et lyophilisées. Le lyophilisât obtenu a été dissout dans 10 % d'acide heptafluorobutyrique et injecté par fraction de 500 µl sur une colonne en phase inverse semi-préparative ultrashere ODS C18 7 µ 250 X 10 mm (Beckman). L'élution des molécules de Pyr-gal-glc a été effectuée à un débit de 3 ml/minute avec une solution à 17 % d'acétonitrile et 0,15 % d'acide haptafluorobutyrique. Les molécules de Pyr-gal-glc éluées entre 15 et 20 minutes ont été collectées, lyophilisées et conservées à - 30°C. Toutes ces opérations ont été répétées afin de traiter les 50 litres d'urines d'enfants.

### - Caractérisation par spectrométrie de masse

Un aliquote de 20 mg de pyr-gal-g1c est passé sur une colonne échangeuse de cation (cellulose phosphate cation exchanger, Sigma), afin d'éliminer l'acide hepafluorobutyrique.

Pour la caractérisation par spectrométrie de masse, il suffit de se reporter aux techniques générales de spectrométrie de masse.

### Résultats :

La méthode de purification de la pyridinoline diglycosylée décrite dans cet exemple est particulièrement avantageuse car l'étape de passage sur cellulose est réalisée sur un entonnoir filtrant au lieu d'une colonne de cellulose et est de plus réalisée avant l'étape de gel filtration. L'utilisation d'un entonnoir filtrant retenant la cellulose pour l'étape de passage sur cellulose et/ou la réalisation de l'étape de gel filtration après l'étape de passage sur cellulose sont avantageuses car ces caractéristiques permettent séparément ou en combinaison une purification rapide et une facilité d'exécution de la méthode. De telles caractéristiques permettent un gain de temps important (environ une semaine pour la purification de 50 1 d'urine) par rapport à des techniques de purification qui ne présentent pas de telles caractéristiques.

La pyridinoline diglycosylée obtenue en quantité suffisante peut être utilisée comme immunogène pour la production d'anticorps monoclonaux.

### Exemple 4 : étude clinique sur l'excrétion urinaire des pyridinolines chez des femmes pré- et post- ménopausées et chez des patients présentant une maladie de Paget ou une polyarthrite rhumatoïde

### Méthodes :

L'excrétion urinaire des pyridinoline et deoxypyridinoline totales a été mesurée par HPLC après hydrolyse acide (ou totale) par HCl 6N. L'excrétion urinaire des formes libres de la pyridinoline (pyridinoline sans peptide) de la deoxypyridinoline libre (deoxypyridinoline sans peptide)a été mesurée par HPLC, sans hydrolyse préalable. La concentration urinaire de CrossLaps (marqueur spécifique de la résorption osseuse) (CrossLaps ELISA, Osteometer Biotech AS), et de CartiLaps (marqueur spécifique de la dégradation du cartilage) (Cartilaps ELISA, Osteometer Biotech AS) ont été analysées par technique ELISA.

Chacun de ces marqueurs a été mesuré chez :
- 40 contrôles (femmes pré-ménopausées et femmes post-ménopausées)
- 27 patients présentant une polyarthrite rhumatoïde (PR)
- 10 patients présentant une maladie de Paget

### Résultats :

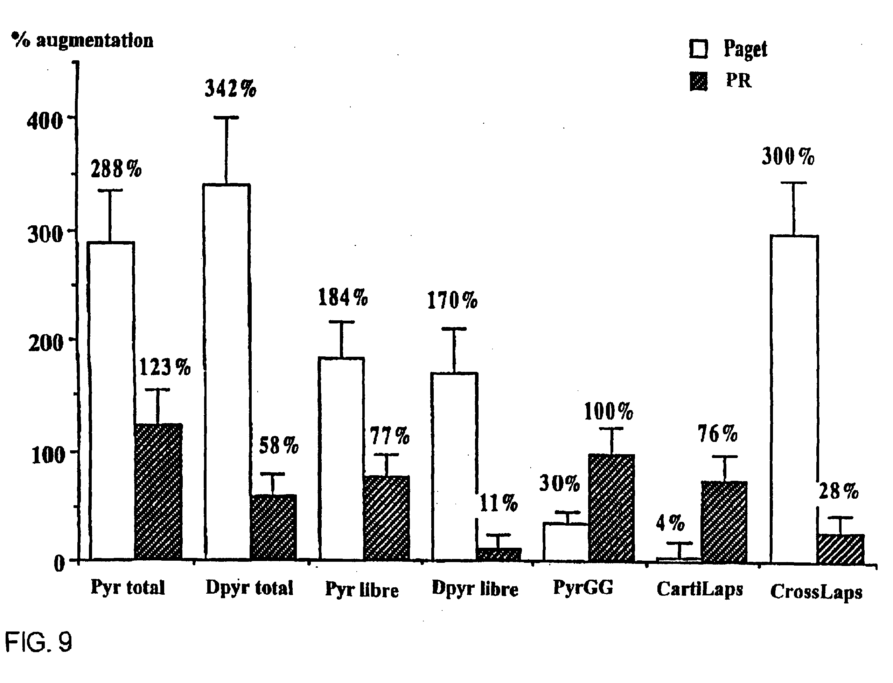

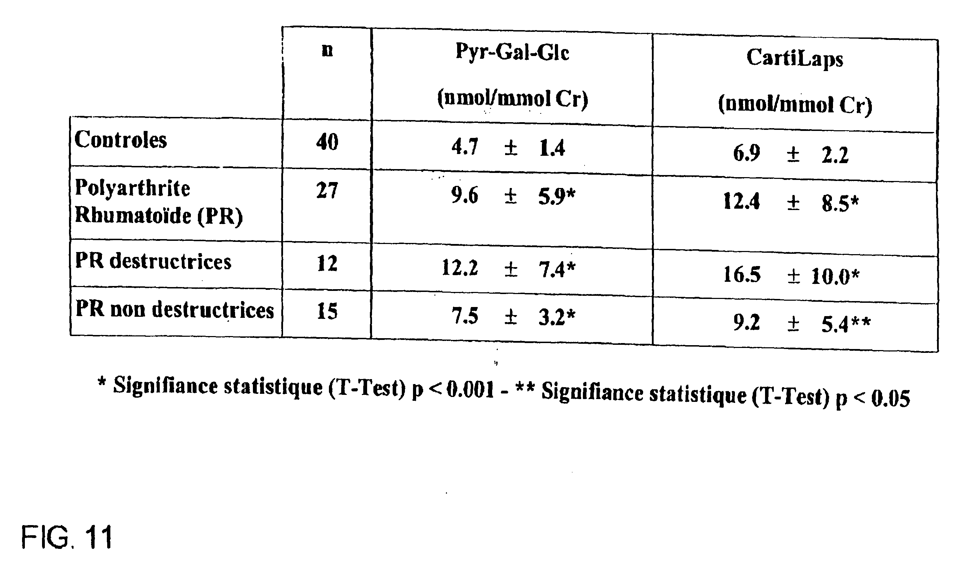

L'excrétion urinaire de la Pyr-gal-glc est significativement augmentée (100%) chez les patients présentant une polyarthrite rhumatoïde (9.6 ± 5.9) comparés au groupe contrôle (4.7 ± 1.4), alors que l'augmentation (30%) observée chez les pagétiques (6.1 ± 1.9) n'est pas significative (figures 8 et 9). Par contre les concentrations urinaires des autres marqueurs analysés (Pyr total, DPyr total, Pyr libre, DPyr libre, Crosslaps) sont présents en quantité plus élevée chez les Pagetiques que chez les patients atteints de PR. Seule l'excrétion urinaire du CartiLaps (marqueur de la dégradation du collagène de type II) est plus élevée chez les PR (76%) comparés au Paget (4%). Il existe une corrélation significative (p= 0.0001) avec un coefficient de corrélation de 0.80 entre les concentrations en Pyr-gal-glc et en CartiLaps (figure 10). Les patients présentant une PR ont été divisés en deux sous groupes: PR destructrices (n = 12) et PR non destructrices (n = 15). L'excrétion urinaire de Pyr-gal-glc est plus élevée dans le groupe des PR destructrices comparé au groupe des PR non destructrices (figure 11). Ces résultats indiquent que des taux augmentés de Pyr-gal-glc sont associés à une destruction articulaire importante.

### Exemple 5 : Etude clinique sur l'excrétion urinaire de Pyr-gal-glc chez des patients atteints de gonarthrose

### Méthode :

L'excrétion urinaire de Pyr-gal-glc a été mesurée par HPLC chez 50 patients présentant une arthrose du genou.

### Résultats :

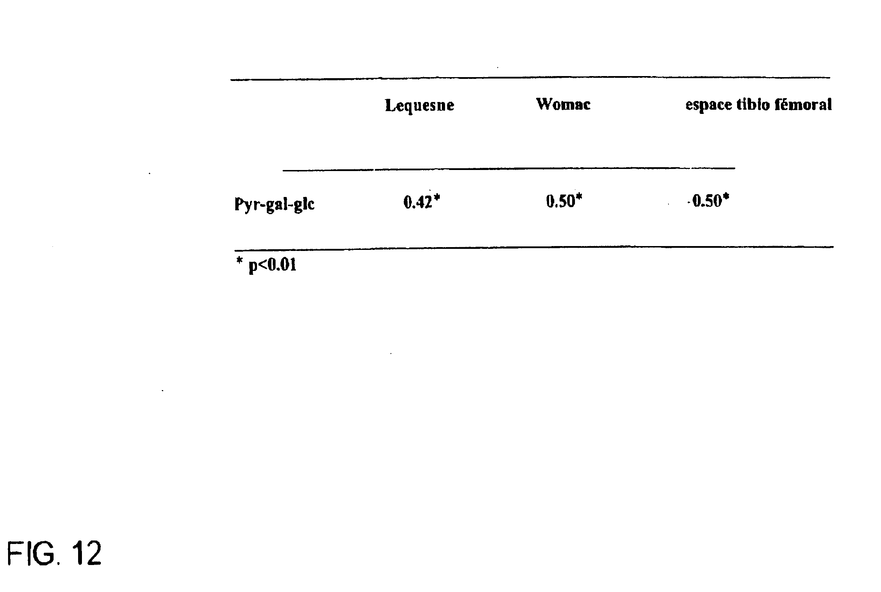

L'excrétion urinaire de Pyr-gal-glc a été corrélée aux indices algofonctionnels de Lequesne [Lequesne M et al. Scandinavian Journal of Rheumatology. (1987) 18 (supplement 65), 85-9] et Womac [Bellamy N et al. Journal of Rheumatology.(1988) 15, 1833-40] et à l'interligne articulaire (figure 12). Les corrélations positives obtenues avec l'indice de Lequesne et l'indice de Womac suggèrent que des taux augmentés de Pyr-gal-glc sont associés à une douleur plus élevée et à une mobilité diminuée. La corrélation négative obtenue avec l'interligne articulaire, indique que des taux augmentés de Pyr-gal-glc sont associés à une destruction cartilagineuse plus importante.

### Exemple 6 : Aspect prédictif du taux de Pyr-Gal-Glc urinaire chez des patients attends de polyarthrite rhumatoïde.

### Méthode :

L'excrétion urinaire de Pyr-Gal-Glc a été mesurée par HPLC chez 112 patients atteints de polyarthrite rhumatoïde depuis moins de trois ans. Le taux mesuré au début de l'expérience est le taux de base permettant les comparaisons.

Le rétrécissement de l'interligne articulaire, l'érosion osseuse et un score total correspondant à la combinaison de ces deux indicateurs sont établis par radiographie des mains et des pieds aux rayons X (score de Sharp) chez tous les patients au début de l'étude, après 6 mois et après un an.

### Résultats :

Les patients atteints de polyarthrite-rhumatoide et à un stade précoce de la maladie présentent des taux de Pyr-Gal-Glc dans leurs urines supérieurs aux 64 contrôle sains de même sexe et de même âge : 7,6 nmol/mmol de créatinine contre 4,4 nmol/mmol de créatinine (p < 0.0001) pour les contrôles.

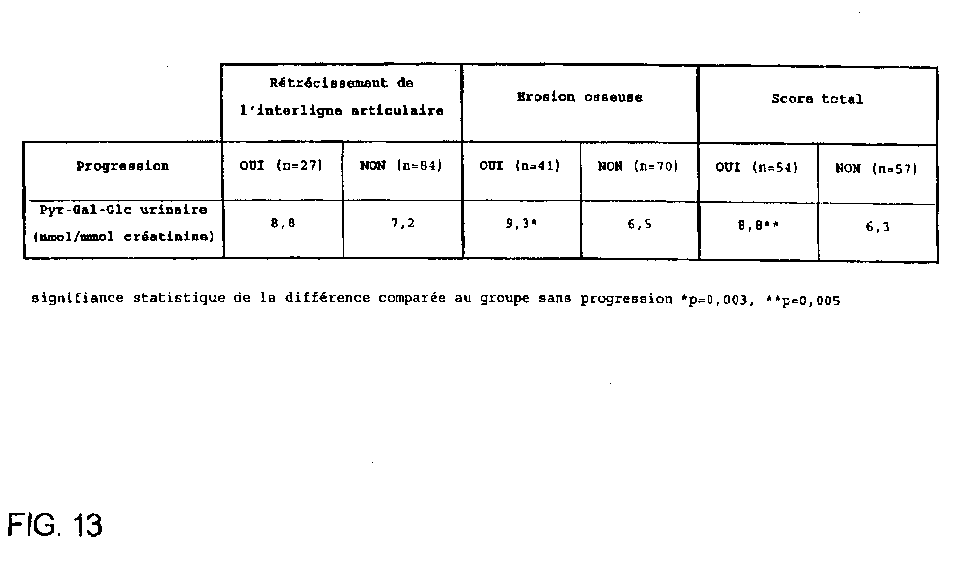

Le tableau de la figure 13 présente les résultats de l'étude longitudinale menée sur un an. Pour les 112 patients, il a été examiné s'ils présentaient une aggravation de la pathologie, sur la base de trois indicateurs, à savoir rétrécissement de l'espace articulaire, érosion osseuse et combinaison des deux précédents (score total). En cas de progression de la destruction articulaire sur la base de l'évolution d'un indicateur, le patient est classé dans la colonne « oui » pour cet indicateur, et dans l'autre colonne dans le cas contraire. L'analyse des résultats fait apparaître clairement que les patients qui présentent par radiographie aux rayons X une progression de la destruction articulaire sur un an, avaient des niveaux de base de Pyr-Gal-Glc urinaires élevées. Les patients dont les niveaux de base de Pyr-Gal-Glc étaient supérieurs de deux écarts types au niveau moyen des contrôles sains (soit 39% de la population) sont sujets à un risque accru de progression de maladie articulaire avec un odds-ratio de 3,6 (1,5-8,1) [intervalle de confiance : 95%]. Les résultats de l'étude indiquent clairement qu'à un niveau élevé de Pyr-Gal-Glc est associé un risque accru de destruction rapide des articulations évaluée sur une année, pour la polyarthrite rhumatoïde à un stade précoce.

## Revendications

1. Méthode pour le diagnostic d'une pathologie synoviale chez un individu susceptible d'être atteint de patologie synoviale **caractérisée par** :
i) la mise en contact in vitro d'un échantillon biologique provenant d'un individu avec un moyen pour mesurer un marqueur spécifique de pathologie synoviale, ledit marqueur spécifique de pathologie synoviale étant la pyridinoline diglycosylée ;
ii) la détermination du niveau du marqueur spécifique ;
iii) la comparaison du niveau du marqueur avec un niveau de référence représentant l'absence de la pathologie, un niveau supérieur du marqueur par rapport au niveau de référence indiquant la présence de la pathologie synoviale.

2. Méthode pour le suivi de l'évolution d'une pathologie ostéoarticulaire **caractérisée par** :
i) la mise en contact in vitro d'un échantillon biologique provenant d'un individu, avec un moyen pour mesurer un marqueur spécifique de pathologie synoviale, ledit marqueur spécifique de pathologie synoviale étant la pyridinoline diglycosylée ;
ii) la détermination du niveau du marqueur spécifique :
iii) la comparaison du niveau du marqueur avec un niveau de référence représentant un stade prédéterminé de la pathologie, un niveau supérieur du marqueur par rapport au niveau de référence indiquant l'évolution de la pathologie ostéoarticulaire.

3. Méthode pour la détermination d'un pronostic d'évolution vers une pathologie ostéoarticulaire ou vers un stade prédéterminé de pathologie ostéoarticulaire
**caractérisée par** :
i) la mise en contact in vitro d'un échantillon biologique provenant d'un individu avec un moyen pour mesurer un marqueur spécifique de pathologie synoviale, ledit marqueur spécifique de pathologie synoviale étant la pyridinoline diglycosylée ;
ii) la détermination du niveau du marqueur spécifique :
iii) la comparaison du niveau du marqueur avec un niveau de référence et la déduction de cette comparaison d'un pronostic d'évolution vers une pathologie ostéoarticulaire ou vers un stade de pathologie ostéoarticulaire

4. Méthode selon l'une des revendications 2 ou 3, **caractérisée en ce que** l'individu est atteint de pathologie ostéoarticulaire ou susceptible d'être atteint de pathologie ostéoarticulaire.

5. Méthode pour la détermination de l'efficacité d'un médicament administré à un individu pour le traitement d'une pathologie ostéoarticulaire **caractérisée par** :
i) la mise en contact in vitro d'un échantillon biologique provenant d'un individu sous traitement, avec un moyen pour mesurer un marqueur spécifique de pathologie synoviale, ledit marqueur spécifique de pathologie synoviale étant la pyridinoline diglycosylée ;
ii) la détermination du niveau du marqueur spécifique :
iii) la comparaison du niveau du marqueur avec un niveau de référence représentant un stade prédéterminé de la pathologie, le niveau du marqueur par rapport au niveau de référence indiquant l'évolution de la pathologie synoviale, et ainsi le degré d'efficacité du traitement

6. Méthode selon l'une des revendications 2 à 5 **caractérisée en ce que** la pathologie ostéoarticulaire est un rhumatisme inflammatoire, une arthropathie métabolique, un rhumatisme dégénératif, la polyarthrite rhumatoïde, une spondylarthropathie, la goutte, la chondrocalcinose ou l'arthrose.

7. Méthode selon l'une des revendications 2 à 6 **caractérisée en ce que** le stade prédéterminé est le stade destructeur ou le stade non destructeur.

8. Méthode selon l'une des revendications 1 à 7 **caractérisée en ce que** la détermination du niveau du marqueur spécifique de pathologie synoviale est effectuée par une technique immunologique, par un immunodosage, par fluorescence, par spectroscopie aux ultraviolets ou par détection électrochimique.

9. Méthode selon la revendication 8 **caractérisée en ce que** la technique immunologique est une technique utilisant des anticorps spécifiques monoclonaux ou polyclonaux, une technique ELISA, une technique immuno-enzymatique, une technique d'immunofluorescence, une technique radio-immunologique ou une technique chemo-immunologique.

10. Méthode selon l'une des revendications 1 à 7 **caractérisée en ce que** la détermination du niveau du marqueur spécifique de pathologie synoviale est effectuée par une technique de chromatographie liquide HPLC.

11. Méthode selon la revendication 10 **caractérisée en ce que** le niveau de référence est choisi dans l'intervalle compris entre environ 5 nmole/mmole Créatinine et environ 9 nmole/mmole Créatinine.

12. Méthode selon l'une des revendications 1 à 11 dans laquelle la détermination du niveau du marqueur spécifique de pathologie synoviale est réalisée dans un échantillon de liquide corporel.

13. Méthode selon la revendication 12 dans laquelle le liquide corporel est choisi parmi le sang, le sérum, le plasma, l'urine, la salive, la sueur ou le liquide synovial.

14. Kit pour le diagnostic, ou le suivi de l'évolution d'une pathologie synoviale **caractérisé en ce qu'**il comprend au moins un moyen pour mesurer un marqueur spécifique de pathologie synoviale, ledit marqueur spécifique de pathologie synoviale étant la pyridinoline diglycosylée, et une mention du niveau de référence représentant l'absence de la pathologie ou représentant un stade prédéterminé de la pathologie.

15. Kit pour le diagnostic, le suivi ou le pronostic de l'évolution d'une pathologie ostéoarticulaire **caractérisé en ce qu'**il comprend au moins un moyen pour mesurer un marqueur spécifique de pathologie synoviale, ledit marqueur spécifique de pathologie synoviale étant la pyridinoline diglycosylée, et une mention du niveau de référence représentant l'absence de la pathologie ou représentant un stade prédéterminé de la pathologie.

16. Kit pour la détermination de l'efficacité d'un médicament administré à un individu pour le traitement d'une pathologie ostéoarticulaire **caractérisé en ce qu'**il comprend au moins un moyen pour mesurer un marqueur spécifique de pathologie synoviale, ledit marqueur spécifique de pathologie synoviale étant la pyridinoline diglycosylée, et une mention du niveau de référence représentant un niveau d'efficacité pour ledit médicament.

17. Utilisation in vitro de pyridinoline diglycosylée comme marqueur spécifique de pathologie synoviale.

18. Utilisation in vitro de pyridinoline diglycosylée comme marqueur spécifique de dégradation de collagène synovial.

## Claims

1. A method for diagnosing a synovial disease in an individual susceptible of developing a synovial disease, **characterized by**:
i) bringing a biological sample from an individual into contact, in vitro, with a means for measuring a specific marker for synovial disease, said specific marker for synovial disease being diglycosylated pyridinoline;
ii) determining the level of the specific marker;
iii) comparing the level of the marker with a reference level representing the absence of the disease, an increased level of the marker with respect to the reference level indicating the presence of the synovial disease.

2. A method for monitoring the evolution of an osteoarticular disease, **characterized by**:
i) bringing a biological sample from an individual into contact, in vitro, with a means for measuring a specific marker for synovial disease, said specific marker for synovial disease being diglycosylated pyridinoline;
ii) determining the level of the specific marker;
iii) comparing the level of the marker with a reference level representing a predetermined stage in the disease, an increased level of the marker with respect to the reference level indicating the evolution of the osteoarticular disease.

3. A method for determining a prognosis of evolution towards an osteoarticular disease or towards a predetermined stage in osteoarticular disease, **characterized by**:
i) bringing a biological sample from an individual into contact, in vitro, with a means for measuring a specific marker for synovial disease, said specific marker for synovial disease being diglycosylated pyridinoline;
ii) determining the level of the specific marker;
iii) comparing the level of the marker with a reference level and deducing a prognosis of evolution towards an osteoarticular disease or towards a stage in osteoarticular disease from that comparison.

4. A method according to claim 2 or claim 3, **characterized in that** the individual has an osteoarticular disease or is susceptible of developing an osteoarticular disease.

5. A method for determining the efficacy of a drug administered to an individual for the treatment of an osteoarticular disease, **characterized by**:
i) bringing a biological sample from an individual under treatment into contact, in vitro, with a means for measuring a specific marker for synovial disease, said specific marker for synovial disease being diglycosylated pyridinoline;
ii) determining the level of the specific marker;
iii) comparing the level of the marker with a reference level representing a predetermined stage of the disease, the level of the marker with respect to the reference level indicating the evolution of the synovial disease, and therefore the degree of efficacy of the treatment.

6. A method according to any one of claims 2 to 5, **characterized in that** the osteoarticular disease is inflammatory rheumatism, a metabolic arthropathy, degenerative rheumatism, rheumatoid arthritis, spondylarthritis, gout, chondrocalcinosis or arthrosis.

7. A method according to any one of claims 2 to 6, **characterized in that** the predetermined stage is the destructive or non destructive stage.

8. A method according to any one of claims 1 to 7, **characterized in that** the specific marker for synovial disease is determined by an immunological technique, by immunoassay, by fluorescence, by ultraviolet spectroscopy or by electrochemical detection.

9. A method according to claim 8, **characterized in that** the immunological technique is a technique employing specific monoclonal or polyclonal antibodies, an ELISA technique, an immuno-enzymatic technique, an immunofluorescent technique, a radio-immunological technique or a chemo-immunological technique.

10. A method according to any one of claims 1 to 7, **characterized in that** the level of the specific marker for synovial disease is determined by a liquid chromatography HPLC technique.

11. A method according to claim 10, **characterized in that** the reference level is selected to be in the range from about 5 nmole/nmole creatinin to about 9 nmole/nmole creatinin.

12. A method according to any one of claims 1 to 11, in which the level of the specific marker for synovial disease is determined in a sample of body fluid.

13. A method according to claim 12, in which the body fluid is selected from blood, serum, plasma, urine, saliva, sweat or synovial fluid.

14. A kit for diagnosing or monitoring the evolution of a synovial disease, **characterized in that** it comprises at least one means for measuring a specific marker for synovial disease, said specific marker for synovial disease being diglycosylated pyridinoline and mentions the reference level representing the absence of disease or representing a pre-determined stage of the disease.

15. A kit for diagnosing, monitoring or prognosing the evolution of an osteoarticular disease, **characterized in that** it comprises at least one means for measuring a specific marker for synovial disease, said specific marker for synovial disease being diglycosylated pyridinoline and mentions the reference level representing the absence of disease or representing the absence of the disease or representing a predetermined stage in the disease.

16. A kit for determining the efficacy of a drug administered to an individual for the treatment of an osteoarticular disease, **characterized in that** it comprises at least one means for measuring a specific marker for synovial disease, said specific marker for synovial disease being diglycosylated pyridinoline and mentions a reference level representing a degree of efficacy of the drug.

17. Use in vitro of diglycosylated pyridinoline as a specific marker for synovial disease.

18. Use in vitro of diglycosylated pyridinoline as a specific marker for synovial collagen degradation.

## Patentansprüche

1. Verfahren zur Diagnose einer synovialen Erkrankung bei einer Person, die veranlagt ist, an einer synovialen Erkrankung erkrankt zu sein, **gekennzeichnet durch**:
i) das Inkontaktbringen in vitro einer biologischen Probe, die von einer Person stammt, mit einem Mittel, um einen für die synoviale Erkrankung spezifischen Marker zu messen, wobei der für die synoviale Erkrankung spezifische Marker diglykosyliertes Pyridinolin ist;
ii) die Bestimmung des Werts an spezifischem Marker;
iii) den Vergleich des Werts des Markers mit einem Referenzwert, der das Nichtvorliegen der Erkrankung darstellt, wobei ein höherer Wert des Markers bezogen auf den Referenzwert das Vorliegen der synovialen Erkrankung anzeigt.

2. Verfahren für die Weiterverfolgung der Entwicklung einer osteoartikulären Erkankung, **gekennzeichnet durch**:
i) das Inkontaktbringen in vitro einer biologischen Probe, die von einer Person stammt, mit einem Mittel, um einen für die synoviale Erkrankung spezifischen Marker zu messen, wobei der für die synoviale Erkrankung spezifische Marker diglykosyliertes Pyridinolin ist;
ii) die Bestimmung des Werts an spezifischem Marker;
iii) den Vergleich des Werts des Markers mit einem Referenzwert, der ein vorbestimmtes Stadium der Erkrankung darstellt, wobei ein höherer Wert des Markers bezogen auf den Referenzwert die Entwicklung der osteoartikulären Erkrankung anzeigt.

3. Verfahren zur Bestimmung einer Prognose der Entwicklung zu einer osteoartikulären Erkrankung oder zu einem vorbestimmten Stadium der osteoartikulären Erkrankung, **gekennzeichnet durch**:
i) das Inkontaktbringen in vitro einer biologischen Probe, die von einer Person stammt, mit einem Mittel, um einen für die synoviale Erkrankung spezifischen Marker zu messen, wobei der für die synoviale Erkrankung spezifische Marker diglykosyliertes Pyridinolin ist;
ii) die Bestimmung des Werts an spezifischem Marker;
iii) den Vergleich des Werts des Markers mit einem Referenzwert und Ableiten einer Prognose der Entwicklung zu einer osteoartikulären Erkrankung oder zu einem Stadium der osteoartikulären Erkrankung aus diesem Vergleich.

4. Verfahren gemäß einem der Ansprüche 2 oder 3, **dadurch gekennzeichnet, dass** die Person an der osteoartikulären Erkrankung erkrankt ist oder veranlagt ist, an der osteoartikulären Erkrankung zu erkranken.

5. Verfahren zur Bestimmung der Wirksamkeit eines einer Person verabreichten Medikaments für die Behandlung einer osteoartikulären Erkrankung, **gekennzeichnet durch**:
i) das Inkontaktbringen in vitro einer biologischen Probe, die von einer sich in Behandlung befindlichen Person stammt, mit einem Mittel, um einen für die synoviale Erkrankung spezifischen Marker zu messen, wobei der für die synoviale Erkrankung spezifische Marker diglykosyliertes Pyridinolin ist;
ii) die Bestimmung des Werts an spezifischem Marker;
iii) den Vergleich des Werts des Markers mit einem Referenzwert, der ein vorbestimmtes Stadium der Erkrankung darstellt, wobei der Wert des Markers bezogen auf den Referenzwert die Entwicklung der synovialen Erkrankung sowie den Grad der Wirksamkeit der Behandlung anzeigt.

6. Verfahren gemäß einem der Ansprüche 2 bis 5, **dadurch gekennzeichnet, dass** die osteoartikuläre Erkrankung entzündliches Rheuma, metabolische Arthropathie, degeneratives Rheuma, rheumatische Polyarthritis, Spondylarthropathie, Gicht, Chondrokalzinose oder Arthrose ist.

7. Verfahren gemäß einem der Ansprüche 2 bis 6, **dadurch gekennzeichnet, dass** das vorbestimmte Stadium das zerstörende Stadium oder das nicht zerstörende Stadium ist.

8. Verfahren gemäß einem der Ansprüche 1 bis 7, **dadurch gekennzeichnet, dass** die Bestimmung des Werts des für die synoviale Erkrankung spezifischen Markers mittels einer immunologischen Technik, mittels Immunoassay, mittels Fluoreszenz, mittels UV-Spektroskopie oder mittels elektrochemischer Detektion durchgeführt wird.

9. Verfahren gemäß Anspruch 8, **dadurch gekennzeichnet, dass** die immunologische Technik eine Technik, bei der spezifische monoklonale oder polyklonale Antikörper verwendet werden, eine ELISA-Technik, eine immuno-enzymatische Technik, eine Immunofluoreszenz-Technik, eine radioimmunologische Technik oder eine chemoimmunologische Technik ist.

10. Verfahren gemäß einem der Ansprüche 1 bis 7, **dadurch gekennzeichnet, dass** die Bestimmung des Werts des für die synoviale Erkrankung spezifischen Markers mittels einer HPLC-Flüssigchromatographietechnik durchgeführt wird.

11. Verfahren gemäß Anspruch 10, **dadurch gekennzeichnet, dass** der Referenzwert in einem Bereich gewählt wird, der zwischen ungefähr 5 nmol/mmol Creatinin und ungefähr 9 nmol/mmol Creatinin liegt.

12. Verfahren gemäß einem der Ansprüche 1 bis 11, bei welchem die Bestimmung des Werts des für die synoviale Erkrankung spezifischen Markers in einer Probe von Körperflüssigkeit durchgeführt wird.

13. Verfahren gemäß Anspruch 12, bei welchem die Körperflüssigkeit aus Blut, Serum, Plasma, Urin, Speichel, Schweiß oder synovialer Flüssigkeit gewählt wird.

14. Kit für die Diagnose oder die Weiterverfolgung der Entwicklung einer synovialen Erkrankung, **dadurch gekennzeichnet, dass** es wenigstens ein Mittel zum Messen eines für die synoviale Erkrankung spezifischen Markers aufweist, wobei der für die synoviale Erkrankung spezifische Marker diglykosyliertes Pyridinolin ist, sowie eine Angabe über den Referenzwert, welcher das Nichtvorliegen der Erkrankung oder ein vorbestimmtes Stadium der Erkrankung darstellt.

15. Kit für die Diagnose, die Weiterverfolgung oder die Prognose der Entwicklung einer osteoartikulären Erkrankung, **dadurch gekennzeichnet, dass** es wenigstens ein Mittel zum Messen eines für die synoviale Erkrankung spezifischen Markers aufweist, wobei der für die synoviale Erkrankung spezifische Marker diglykosyliertes Pyridinolin ist, sowie eine Angabe über den Referenzwert, welcher das Nichtvorliegen der Erkrankung oder ein vorbestimmtes Stadium der Erkrankung darstellt.

16. Kit für die Bestimmung der Wirksamkeit eines einer Person verabreichten Medikaments für die Behandlung einer osteoartikulären Erkrankung, **dadurch gekennzeichnet, dass** es wenigstens ein Mittel zum Messen eines für die synoviale Erkrankung spezifischen Markers aufweist, wobei der für die synoviale Erkrankung spezifische Marker diglykosyliertes Pyridinolin ist, sowie eine Angabe über den Referenzwert, welcher einen Grad der Wirksamkeit des Medikaments darstellt.

17. Verwendung in vitro von diglykosyliertem Pyridinolin als für die synoviale Erkrankung spezifischer Marker.

18. Verwendung in vitro von diglykosyliertem Pyridinolin als für den Abbau synovialen Kollagens spezifischer Marker.
